(19) Europäisches Patentamt
European Patent Office
Office européen des brevets

(11) Publication number: **0 391 390 B1**

(12) **EUROPEAN PATENT SPECIFICATION**

(45) Date of publication of patent specification: **08.02.95**

(51) Int. Cl.[6]: **C07D 237/16**, C07D 237/18, C07D 237/22, A01N 43/58

(21) Application number: **90106436.0**

(22) Date of filing: **04.04.90**

The file contains technical information submitted after the application was filed and not included in this specification

(54) **Pyridazinones having insecticidal and acaricidal activity and compositions containing them.**

(30) Priority: **05.04.89 IT 2001389**

(43) Date of publication of application:
**10.10.90 Bulletin 90/41**

(45) Publication of the grant of the patent:
**08.02.95 Bulletin 95/06**

(84) Designated Contracting States:
**AT BE CH DE ES FR GB LI NL**

(56) References cited:
**EP-A- 0 088 384**
**EP-A- 0 134 439**
**EP-A- 0 183 212**
**EP-A- 0 199 281**
**EP-A- 0 210 647**
**EP-A- 0 232 825**
**EP-A- 0 283 271**

(73) Proprietor: **MINISTERO DELL' UNIVERSITA' E DELLA RICERCA SCIENTIFICA E TECNOLOGICA**
**76, Lungotevere Thaon de Revel**
**I-00196 Roma (IT)**

(72) Inventor: **Bettarini, Franco**
**4/B, via Cadore**
**I-28100 Novara (IT)**
Inventor: **Capuzzi, Luigi**
**23, via Rivolta**
**I-28100 Novara (IT)**
Inventor: **Massimini, Sergio**
**15, via Mercantini**
**I-20158 Milan (IT)**
Inventor: **Castoro, Paolo**
**16, via Birago**
**I-13100 Vercelli (IT)**
Inventor: **Caprioli, Vincenzo**
**8, via Loriga**
**I-27028 S. Martino Siccomario,**
**Pavia (IT)**

(74) Representative: **Barz, Peter, Dr. et al**
**Patentanwälte Dipl.-Ing. G. Dannenberg**
**Dr. P. Weinhold, Dr. D. Gudel**
**Dipl.-Ing. S. Schubert, Dr. P. Barz**
**Siegfriedstrasse 8**
**D-80803 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to derivatives of 3(2H)-pyridazinone, to processes for preparing them, to insecticidal and acaricidal compositions containing them, and to the use of said compositions for the control of insects, mites and ticks.

Compounds belonging to the class of 3(2H)-pyridazinones and displaying fungicidal, insecticidal, acaricidal and nematocidal activity have been disclosed in EP-A-88,384; 134,439; 183,212; 199,281; 232,825.

The present invention in particular relates to a novel class of substituted pyridazinones which show an increased activity in the control of harmful insects and mites, in particular of harmful mites in the agricultural, civil and zootechnical field.

In fact, pyridazinones of general formula (I), as defined herein and containing in their molecule specific halogenated moieties of formula

$-(Y_1)_y-(W-O)_w-R_x$

linked as substituents to an aromatic ring, are endowed with a very high acaricidal activity, higher than that of compounds of the prior art in which corresponding radicals do not contain halogen atoms.

Therefore, the object of the present invention are novel pyridazinones having the general formula (I):

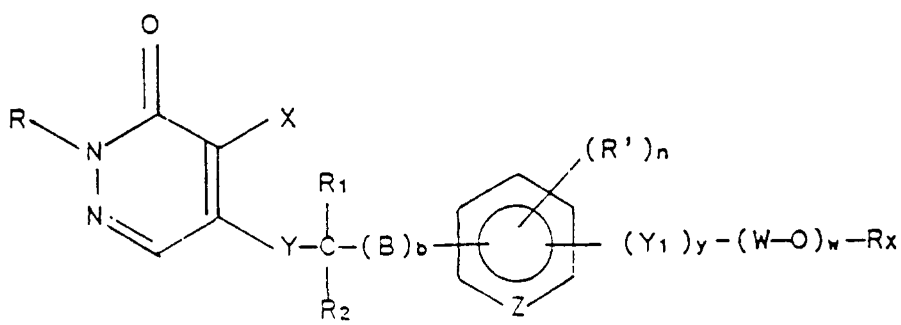

(I)

wherein:

| | |
|---|---|
| R | represents a linear or branched ($C_2$-$C_6$) alkyl group or a phenyl group, optionally substituted by one or more halogen atoms and/or lower alkyl, haloalkyl, alkoxy and/or haloalkoxy radicals; (the term "lower" as used herein denotes groups having 1 to 6, particularly 1 to 4 carbon atoms); |
| X | represents a halogen atom (F, Cl, Br or I); |
| Y | represents O, S or NH; |
| B | represents 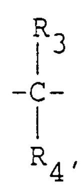 |
| b | is either O or 1; |
| $R_1$, $R_2$, $R_3$ and $R_4$ | independently represent H or a linear or branched ($C_1$-$C_4$) alkyl or haloalkyl radical; |
| Z | represents CH or N, preferably CH; |
| $Y_1$ | represents O, S, CO or |

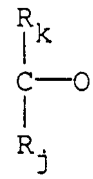

wherein:

$R_k$ and $R_j$ independently are H or a linear or branched ($C_1$-$C_4$) alkyl or haloalkyl radical;

- W is a linear or branched ($C_2$-$C_6$) haloalkylene group;
- y and w independently are 0 or 1;
- $R_x$ for w = O represents a linear or branched ($C_2$-$C_6$) haloalkenyl group optionally having one or more $C_1$-$C_3$ haloalkoxy and/or phenyl groups as substituents, said phenyl group in turn being optionally substituted by one or more halogen atoms and/or ($C_1$-$C_6$) alkyl, haloalkyl, alkoxy and/or haloalkoxy radicals; or represents a -($C_3$-$C_6$) halocycloalkyl or ($C_4$-$C_7$) halocycloalkylalkyl radical, which radicals may have one or more substituents selected from ($C_1$-$C_4$) alkyl or haloalkyl groups; and, for w = 1, represents a linear or branched ($C_1$-$C_6$) haloalkyl radical;
- R' represents a halogen atom (F, Cl, Br or I); and
- n is O, 1 or 2.

When n is 2, the radicals R' can be the same or different from one another.

Preferred compounds are those having formula (Ia):

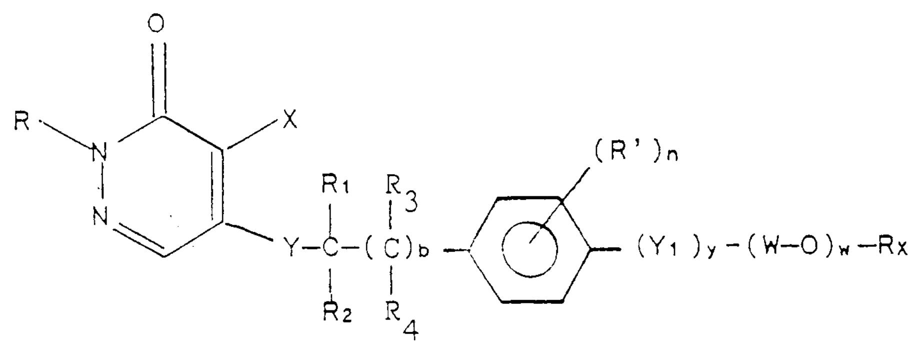

(Ia)

wherein:

- R represents a ($C_2$-$C_6$) alkyl radical;
- Y represents O or S;

and the other symbols have the same meaning as defined hereinabove.

Examples and preferred embodiments of the various generic groups given above are:

$C_{1-6}$, preferably $C_{1-4}$, alkyl groups: methyl, ethyl, n- and i-propyl, n-, i-, sek- and tert.butyl, hexyl;

$C_{1-6}$-, preferably $C_{1-4}$-haloalkyl groups: the above alkyl groups substituted with one or more (up to the maximum) halogen atoms, particularly Cl and/or F;

$C_{1-6}$-, preferably $C_{1-4}$-alkoxy and -haloalkoxy groups:

alkoxy groups derived from the above alkyl and haloalkyl groups;

$C_{2-6}$-, preferably $C_{2-4}$-haloalkylene groups: ethylene, propylene, methylethylene, butylene and hexylene, substituted with one or more (up to the maximum) halogen atoms, particularly Cl and/or F;

$C_{3-6}$-halocycloalkyl: cyclopropyl, cyclobutyl, cyclopentyl and cyclohexyl, substituted with one or more (up to the maximum) halogen atoms, particularly Cl and/or F;

$C_{4-7}$-halocycloalkylalkyl: the above $C_{3-6}$-halocycloalkyl groups, attached to a $C_{1-3}$-alkylene group, e.g., a methylene group;

$C_{2-6}$-, preferably $C_{2-4}$-haloalkenyl groups: ethenyl, propenyl and butenyl, substituted with one or more (up to the maximum) halogen atoms, particularly Cl and/or F;

substituted phenyl groups preferably are phenyl groups having 1 to 3, particularly 1 or 2, substituents.

Preferred meanings of the various symbols given above are as follows:

- R: $C_{1-4}$-alkyl, particularly (tert.-)butyl; phenyl, optionally substituted with 1 to 3, particularly 1 or 2 groups selected from F, Cl, $CH_3$, $C_2H_5$, $CF_3$, $C_2F_5$, $CH_3O$,

| | |
|---|---|
| | $C_2H_5O$, $CF_3O$ and $C_2F_5O$; |
| X: | F, Cl or Br, particularly Cl; |
| Y: | O or S; |
| b: | O |
| $R_1$, $R_2$, $R_3$ and $R_4$: | H, $CH_3$, $C_2H_5$, $CCl_3$, $CF_3$ |
| Z: | CH |
| $Y_1$: | O, S, CO, $CH_2O$, $CH_2CH_2O$, $CH(CH_3)O$, $C(CH_3O)_2O$, $CH(CF_3)O$; |
| W: | $CF_2CF_2$, $CHFCF_2$, $CHClCF_2$, $CCl_2CF_2$, CHFCHF, CHClCHF, $CHFCCl_2$ and other ethylene groups substituted with one or more (up to the maximum) F and/or Cl atoms; |
| $R_x$: | for w = O, in general a $C_{2-4}$-alkenyl group, particuarly -CH =CH - wherein at least one of the hydrogen atoms is replaced by Cl and/or F and other hydrogen atoms may be replaced by one or more groups selected from $CF_3O$, $C_2F_5O$, $C_3F_7O$ and chlorinated analogues thereof, phenyl and phenyl having up to 5 halogen atoms (particularly Cl and/or F) and/or $CF_3$ and $CF_3O$ groups; cyclopropyl and cyclopropylmethyl wherein one or more hydrogen atoms in the ring are replaced by F, Cl and/or $CH_3$; specific examples of preferred groups $R_x$ are the following: |
| w = O: | $CF_3CCl=CH$, $CF_3CF=CH$, $CF_3CF=CF$, $CF_3O$-CF=CF, $CF_3O$-CCl=CH, $CF_3O$-CF=CH, $C_2F_5O$-CF=CF, $C_2F_5O$-CCl=CH, $C_2F_5O$-CF=CH, $Cl_2C=CH$, $Cl_2C=CCl$, $Cl_2C=CF$, $F_2C=CH$, $F_2C=CCl$, $F_2C=CF$, $C_6H_5$-CCl=CH, $C_6H_5$-CF=CH, $C_6H_4Cl$-CCl=CH, $C_6H_4Cl$-CF=CH, cyclo-$C_3H_3Cl_2$, cyclo-$C_3H_2(CH_3)Cl_2$, cyclo-$C_3H_3Cl_2$-$CH_2$ and cyclo-$C_3H_2(CH_3)Cl_2$-$CH_2$; |
| w = 1: | $CF_3$, $C_2F_5$, $C_3F_7$ and (partly) chlorinated analogues thereof. |

Particularly preferred compounds of general formula (I) are those wherein $R_1$ is H or $C_{1-4}$-alkyl, particularly methyl, $R_2$ is H and b is 0 and/or wherein the moiety $-(Y_1)_y-(W-O)_w-R_x$ contains at least one F and/or Cl atom and particularly is selected from 1,1,2-trifluoro-2-(partly or completely fluorinated $C_{1-4}$-alkoxy)-ethoxy, 2-chloro-2-(partly or completely fluorinated $C_{1-4}$-alkyl)-ethenyl, 2,2-dichloro-ethenyl, 1,2-difluoro-2-(partly or completely fluorinated $C_{1-4}$-alkoxy)-ethenyl, 2-chloro-2-(optionally substituted phenyl)-ethenyl,ω-(2,2-dichloro-1-methyl-cyclopropyl)-$C_{1-4}$-alkoxy and (2,2-dichloro-1-methyl-cyclopropyl)-carbonyl.

The above compounds are endowed with a remarkably high insecticidal and acaricidal activity towards harmful insects and mites in the agricultural, civil and zootechnical fields. In particular, they are active against important species of the classes Hemiptera, Lepidoptera, Coleoptera, Dipterans, Blattidae, Tetranychidae and Ixodides.

The compounds having the general formula (I) may be prepared by reacting a pyridazinone of formula (II) with a compound of formula (III) according to the reaction scheme (1):

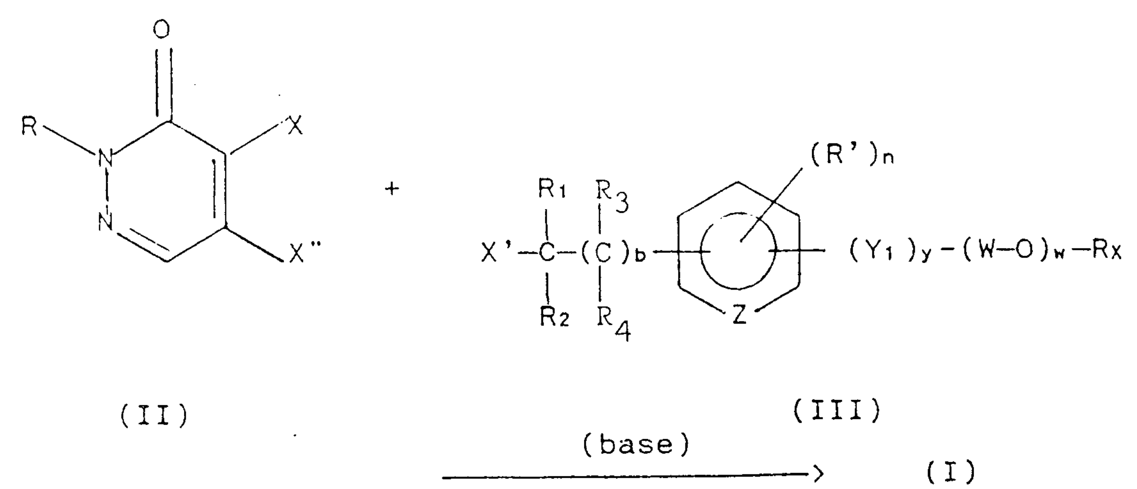


In the above formulae the symbols R, X, $R_1$, $R_2$, $R_3$, $R_4$, Z, $Y_1$, W, $R_x$, R', b, y, w, and n have the same meanings as indicated above. X' and X'' represent Cl, Br or I atoms, or a radical YH, Y having the same meaning as given above, with the proviso that one of X' and X'' is YH while the other one is Cl, Br, or I.

The reaction preferably is carried out in an inert organic solvent such as, e.g., benzene, toluene, acetone, methylethyl-ketone, acetonitrile, dioxane, N,N-dimethyl-formamide and dimethyl-sulfoxide, in the presence of an inorganic base, such as, e.g., sodium hydride, sodium hydroxide, potassium hydroxide, calcium hydroxide, sodium carbonate and potassium carbonate, or of an organic base, such as, e.g., triethylamine or pyridine, and at a temperature of from room temperature to the boiling temperature of the solvent used in the reaction.

The compounds of formula (II) are described in the literature. They can be prepared according to the methods disclosed in, e.g., A.R. Katritzky, A.J. Boulton (Editors), "Advances in Heterocyclic Chemistry", vol, 9, pages 235-236 and 249-258, Academic Press, New York and London 1968, and in the references given therein.

The intermediates of formula (III) are either known or can be prepared by conventional methods of organic chemistry.

The compounds of general formula (I) can be mixtures of isomers whose separation can be carried out by using well known techniques, such as column chromatography or thin-layer chromatography.

The isolation and the use of each individual isomer, as well as the direct use of the mixtures which can be obtained from the preparation of the compounds, and the use of the mixtures deriving from an incomplete separation of the isomers are within the scope of the present invention.

As mentioned above, the compounds of general formula (I) in particular are endowed with high insecticidal and acaricidal activity, in general at all stages of the life cycle of insects and mites (larvae, adult, insects and eggs). They display a wide range of action and an excellent residual activity.

Thanks to their advantageous characteristics, the compounds of formula (I) are suitable for use in controlling harmful insects and mites both in cultivations of agricultural and horticultural interest and premises frequented by man and domestic and breeding animals.

For the purpose of their practical use, both in agriculture and in other sectors, the compounds according to the present invention are advantageously used in the form of suitable compositions.

These compositions may contain, besides one or more compound(s) of formula (I) as their active principle, inert, solid vehicles (such as, e.g., kaolin, silica, talc, attapulgite, bentonite, diatomaceous earth, etc.); or liquid vehicles (organic solvents, vegetable or mineral oils, water and mixtures thereof) and, optionally, further conventional additives such as surfactants, suspending agents, dispersing agents and wetting agents.

For special application requirements or in order to extend the range of action of the compositions, further active ingredients such as, e.g., other insecticides or acaricides, herbicides, fungicides or fertilizers, may be incorporated in said compositions.

The application dosages vary as a function of several factors, among which the type and level of infestation, the type of composition used and climatic and environmental factors may be mentioned.

For use in agriculture doses of the compounds of formula (I) of from 5 g to 5 kg per hectare usually provide a sufficiently high protection.

The following examples are given in order to illustrate the present invention.

Example 1

Synthesis of 4-(1,1,2-trifluoro-2-trifluoromethoxy-ethoxy)-α-methylbenzyl alcohol [Intermediate of formula (III)]

13.6 g of 4-hydroxyacetophenone (100 mmol), 5.6 g of ground potassium hydroxide, 120 ml of toluene and 30 ml of dimethylsulfoxide were placed in a 250 ml-flask.

The flask was evacuated and 16.6 g (100 mmol) of perfluorovinylmethylether ($CF_3OCF=CF_2$) were bubbled through the reaction mixture, the temperature being kept constant at 20 °C.

At the end of the bubbling step, the reaction mixture was acidified, extracted with ether and washed with brine. The ether phases were thoroughly dried over sodium sulfate and were evaporated to dryness on a rotary evaporator. The crude reaction product (25 g) thus obtained was dissolved in 200 ml of anhydrous ethanol. The solution was cooled to 0 °C and 3.8 g of sodium borhydride were added portionwise.

The solution was left standing at room temperature and 30 minutes later was neutralized with a saturated solution of ammonium chloride and thereafter extracted with ether. The ether phases were thoroughly dried and evaporated to dryness and the residues were chromatographed on silica gel, a 9:1 mixture of hexane/ethyl acetate being used as eluent. The eluate then was distilled under vacuum (b.p. 74-79 °C at 0.2 mmHg).

Thus 18.3 g of pure product were obtained.

N.M.R. (60 MHz, $CDCl_3$): δ 1.2 (d, 3H); 3.15 (s, 1H); 4.65 (q, 1H); 5.9 (dt, 1H); 7.25 (ab q, 4H).

Example 2

Synthesis of 4-(1,1,2-trifluoro-2-trifluoromethoxy-ethoxy)-α-methylbenzyl bromide [Intermediate of formula (III)]

3 g of 4-(1,1,2-trifluoro-2-trifluoromethoxy-ethoxy)-α-methylbenzyl alcohol (prepared as described in Example 1) were dissolved in 20 ml of anhydrous ether and the resulting solution was cooled to -70 °C. Then, 0.4 ml of $PBr_3$ were added to the mixture.

The reaction mixture was left standing at room temperature and 30 minutes later a saturated solution of sodium bicarbonate was added thereto. The reaction mixture was extracted with ether and the ether phases were washed with water to neutrality. The ether phases were thoroughly dried, concentrated to dryness by means of a rotary evaporator and chromatographed on silica gel with a 95/5 mixture of hexane/ethyl acetate. Thus 2.2 g of pure product were obtained.

N.M.R. (60 MHz, $CDCl_3$): δ 1.2 (d, 3H); 4.5 (q, 1H); 5.9 (dt, 1H); 7.25 (ab q, 4H).

Example 3

Synthesis of 4-(1,1,2-trifluoro-2-trifluoromethoxy-ethoxy)-benzyl alcohol [Intermediate of formula (III)]

12.4 g of 4-hydroxybenzaldehyde (100 mmol), 5.6 g of ground potassium hydroxide, 120 ml of toluene and 30 ml of dimethylsulfoxide were placed in a 250 ml-flask.

The flask was evacuated and 16.6 g (100 mmol) of perfluoromethylvinylether ($CF_3OCF=CF_2$) were bubbled through the reaction mixture, the temperature being kept constant at 20 °C.

At the end of the bubbling step, the reaction mixture was acidified, extracted with ether and washed with brine. The ether phases were thoroughly dried over sodium sulfate and concentrated to dryness on a rotary evaporator. The crude reaction product (24 g) thus obtained was dissolved in 200 ml of anhydrous ethanol, the resulting solution was cooled to 0 °C and 3.8 g of sodium borhydride were added portionwise.

The solution was left standing at room temperature and 30 minutes later was neutralized with a saturated solution of ammonium chloride and extracted with ether. The ether phases were thoroughly dried, evaporated and chromatographed on silica gel, a 9:1 mixture of hexane/ethyl acetate being used as eluent. The eluate then was distilled under vacuum (b.p. 83 °C at 0.3mmHg).

Thus 14.3 g of pure product were obtained.

N.M.R. (60 MHz, $CDCl_3$): δ 3.15 (s, 1H); 4.65 (s, 1H); 5.9 (dt, 1H); 7.25 (ab q, 4H).

Example 4

Synthesis of 4-(1,1,2-trifluoro-2-trifluoromethoxy-ethoxy)-benzyl bromide [Intermediate of formula (III)]

3 g of 4-(1,1,2-trifluoro-2-trifluoromethoxy-ethoxy)-benzyl alcohol (prepared as described in Example 3) were dissolved in 20 ml of anhydrous ether and the resulting solution was cooled to -70 °C. Then, 0,4 ml of $PBr_3$ were added to the mixture.

The reaction mixture was left standing at room temperature and 30 minutes later a saturated solution of sodium bicarbonate was added thereto. The reaction mixture was extracted with ether and the ether phases were washed with water to neutrality. The ether phases were thoroughly dried, concentrated to dryness on a rotary evaporator and chromatographed on silica gel with a 95/5 mixture of hexane/ethyl acetate. Thus 2.2 g of pure product were obtained.

N.M.R. (60 MHz, $CDCl_3$): δ 4.4 (s, 2H); 5.9 (dt, 1H); 7.3 (ab q, 4H).

Example 5

Synthesis of 4-(2-chloro-3,3,3-trifluoroprop-1-enyl)-benzyl bromide [Intermediate of formula (III)]

12 g of p-tolualdehyde, dissolved in 20 ml of DMF, and 7.5 g of Zn were placed in a 250 ml-flask. 2.5 g of $CF_3CCl_3$ were added dropwise and the resulting reaction mixture was slightly heated until the reaction became strongly exothermic. Then 10 g of $CF_3CCl_3$, dissolved in 100 ml of DMF, were added dropwise and the resulting mixture was stirred at room temperature for 4 hours. Then 10 ml of acetic anhydride and 13 g

of Zn were added. After 2 hours at room temperature, the reaction mixture was poured into acidified water and was extracted three times with ether. The ether phases were combined and washed with a saturated solution Of sodium bicarbonate and with brine, and were then thoroughly dried and evaporated. The crude reaction product was chromatographed on silica (98/2 hexane/ethyl acetate), whereby 11.5 g of 4-(2-chloro-3,3,3-trifluoroprop-1-enyl)-toluene were obtained.

The obtained product was dissolved in 120 ml of $CCl_4$ and 10.8 g of N-bromo-succinimide and 0.5 g of benzoyl peroxide were added. The resulting mixture was stirred under reflux for 2 hours, diluted with hexane, filtered and evaporated. The crude reaction product was chromatographed on silica (99/1 hexane/ethyl acetate), whereby 9 g of product were obtained.

N.M.R (60 MHz, $CDCl_3$): $\delta$ 4.5 (s, 2H); 7.85 - 7.3 (m, 5H).

Example 6

Synthesis of 2-tert.-butyl-4-chloro-5-[4-(1.1.2-trifluoro-2-trifluoromethoxy-ethoxy)-α-methyl-benzyloxy]-3(2H)-pyridazinone [Compound (1)]

1.5 g of 4-(1,1,2-trifluoromethoxy-ethoxy)-α-methylbenzyl alcohol (Example 1) were added to a suspension of 0.24 g of sodium hydride (50% in 10 ml of formaldehyde) and 15 minutes later 1.1 g of 2-tert.-butyl-4,5-dichloro-3(2H)-pyridazinone were added to the resulting mixture. The reaction mixture was stirred for 1 hour at room temperature, diluted with ether and washed with diluted HCl and brine. The washed solution was thoroughly dried, the solvent was evaporated and the crude reaction product thus obtained was chromatographed on silica gel with 9/1 hexane/ethyl acetate.

Thus 0.9 g of pure product were obtained.

N.M.R. (60 MHz, $CDCl_3$): 1.6 (s, 9H); 1.75 (d, 3H); 5.5 (q, 1H); 6.0 (tt, 1H); 7.3 (ab q, 4H); 7.5 (s, 1H).

Example 7

Synthesis of 2-tert.-butyl-4-chloro-5-[4-(1,1,2⁻trifluoro-2-trifluoromethoxy-ethoxy)-α-methylbenzylthio]-3(2H)-pyridazinone [Compound (2)]

1.4 g of 4-(1,1,2-trifluoro-2-trifluoromethoxy-ethoxy)-α-methyl-benzyl bromide (Example 2) and 1 g of 2-tert.-butyl-4-chloro-5-mercapto-3(2H)-pyridazinone were added to a suspension of 0.8 g of potassium carbonate in 10 ml of dimethylformamide. The resulting reaction mixture was stirred for 1 hour at room temperature and was diluted with ether, and washed with diluted HCl and brine. The washed solution was thoroughly dried, the solvent was evaporated and the crude reaction product thus obtained was chromatographed on silica gel with 9/1 hexane/ethyl acetate. Thus 0.58 g of pure product were obtained.

N.M.R (60 MHz $CDCl_3$): $\delta$ 1.6 (s, 9H); 1.75 (d, 3H); 4.75 (q, 1H); 6.0 (tt, 1H); 7.4 (ab q, 4H); 7.5 (s, 1H).

Example 8

Synthesis of 2-tert.-butyl-4-chloro-5-[4-(1,1,2-trifluoro-2-trifluoromethoxy-ethoxy)-benzyloxy]-3(2H)-pyridazinone [Compound (3)]

1.16 g of 4-(1,1,2,-trifluoro-2-trifluoromethoxyethoxy)-benzyl alcohol (Example 3) and, 15 minutes later, 0.95 g of 2-tert.-butyl-4,5-dichloro-3(2H)-pyridazinone were added to a suspension of 0.2 g of sodium hydride (50% in 10 ml of dimethylformamdie). The resulting reaction mixture was stirred for 1 hour at room temperature, was diluted with ether and washed with diluted HCl and brine. The washed solution was thoroughly dried, the solvent was evaporated and the crude reaction product thus obtained was chromatographed on silica gel with 9/1 hexane/ethyl acetate.

Thus 1.3 g of pure product were obtained. Melting point 68-69 °C.

N.M.R (60 MHz $CDCl_3$): $\delta$ 1.65 (s, 9H); 5.5 (d, 2H); 6.1 (tt, 1H); 7.5 (ab q, 4H); 7.9 (s, 1H).

Example 9

Synthesis of 2-tert.-butyl-4-chloro-5-[4-(1,1,2-trifluoro-2-trifluoromethoxyethoxy)-benzylthio]-3(2H)-pyridazinone [Compound (4)]

1.4 g of 4-(1,1,2-trifluoro-2-trifluoromethoxy-ethoxy)-benzyl bromide (Example 4) and 1 g of 2-tert.-butyl-4-chloro-5-mercapto-3(2H)-pyridazinone were added to a suspension of 0.8 g of potassium carbonate in 10 ml of dimethylformamide. The resulting reaction mixture was stirred for 1 hour at room temperature, was diluted with ether and washed with diluted HCl and brine. The washed solution was thoroughly dried, the solvent was evaporated and the crude reaction product thus obtained was chromatographed on silica gel with 9/1 hexane/ethyl acetate. Thus 1.4 g of pure product were obtained.

N.M.R (60 MHz $CDCl_3$): $\delta$ 1.6 (s, 9H); 4.75 (s, 2H); 6.0 (tt, 1H); 7.4 (bs, 4H); 7.5 (s, 1H).

Example 10

Synthesis of 2-tert.-butyl-4-chloro-5-[4⁻(2-chloro-3,3,3-trifluoro-prop-1-enyl)-benzyl-thio]-3-(2H)-pyridazinone [Compound 10]

1.2 g of 4-(2-chloro-3,3,3-trifluoroprop-1-enyl)-benzyl bromide and 0.8 g of 2-tert.-butyl-4-chloro-5-mercapto-3(2H)-pyridazinone were added to a suspension of 0.7 g of potassium carbonate in 10 ml of dimethylformamide.

The resulting reaction mixture was stirred for 1 hour at room temperature, was diluted with ether and washed with diluted HCl and brine. The washed solution was thoroughly dried, the solvent was evaporated and the crude reaction product thus obtained was chromatographed on silica gel with 9/1 hexane/ethyl acetate. Thus 0.9 g of pure product were obtained. Melting point = 124 °C.

N.M.R (60 MHz $CDCl_3$): $\delta$ 1.6 (s, 9H); 4.25 (s, 2H); 6.75 (s, 1H); 7.6-7.2 (m, 5H).

Example 11

Synthesis of 2-tert.-butyl-4-chloro-5-[4-(2,2-dichloroethenyl)-benzylthio]-3(2H)-pyridazinone [Compound (11)-]

1.2 g of 4-(2,2-dichloroethenyl)-benzyl bromide and 0.8 g of 2-tert.-butyl-4-chloro-5-mercapto-3(2H)-pyridazinone were added to a suspension of 0.7 g of potassium carbonate in 10 ml of dimethylformamide.

The resulting reaction mixture was stirred for 1 hour at room temperature, was diluted with ether and washed with diluted HCl and brine. The washed solution was thoroughly dried, the solvent was evaporated and the crude reaction product thus obtained was chromatographed on silica gel with 9/1 hexane/ethyl acetate. Thus, 1.13 g of pure product were obtained. Melting point = 105 °C.

N.M.R (60 MHz $CDCl_3$): $\delta$ 1.6 (s, 9H); 4.25 (s, 2H); 7.6-7.0 (m, 6H).

By following the procedure of Examples 6 to 11, the compounds shown in Table 1 were obtained.
In Table 1 four reference compounds which fall within the scope of EP-A-199281 are also given. Said reference compounds are identified as compounds $RC_1$; $RC_2$; $RC_3$ and $RC_4$.

TABLE 1

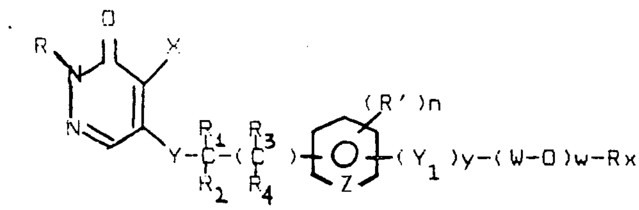

| Comp. | R | X | Y | $C(R_1)(R_2)-(C(R_3)(R_4))$ | Z | (R')n | Rx-(O-W)w(Y1)y | m.p.(°C) |
|---|---|---|---|---|---|---|---|---|
| 1 | t-Bu | Cl | O | CH(Me) | CH | – | $4-CF_3OCFHCF_2O$ | |
| 2 | t-Bu | Cl | S | CH(Me) | CH | – | " | |
| 3 | t-Bu | Cl | O | $CH_2$ | CH | – | " | 68-9 |
| 4 | t-Bu | Cl | S | $CH_2$ | CH | – | " | 98 |
| 5 | t-Bu | Cl | S | $CH_2$ | CH | – | $4-CF_3CF_2OCFHCF_2O$ | 88 |
| 6 | t-Bu | Cl | S | CH(Me) | CH | – | " | |
| 7 | t-Bu | Cl | S | $CH_2$ | CH | – | $4-CF_3OCFHCF_2S$ | 88 |
| 8 | t-Bu | Cl | S | $CH_2$ | CH | – | $4-CF_3OCFHCF_2OCH(Me)$ | 57-9 |
| 9 | t-Bu | Cl | S | $CH_2$ | CH | – | $4-CF_3OCFHCF_2OCH(CF_3)$ | 73-5 |
| 10 | t-Bu | Cl | S | $CH_2$ | CH | – | $4-CF_3C(Cl)=CH$ | 123-5 |
| 11 | t-Bu | Cl | S | $CH_2$ | CH | – | $4-Cl_2C=CH$ | 104-6 |
| 12 | t-Bu | Cl | S | $CH_2$ | CH | – | $4-CF_3C(F)=CH$ | 87-9 |
| 13 | t-Bu | Cl | S | $CH_2$ | CH | – | $4-CF_3C(F)=CF$ | 100-2 |
| 14 | t-Bu | Cl | S | $CH_2$ | CH | – | $4-CF_3OCF=CF$ | 81 |

| | | | | | | | | |
|---|---|---|---|---|---|---|---|---|
| 15 | t-Bu | Cl | S | $CH_2$ | CH | – | 4-$CF_3CF_2OCF$=CF | 70-2 |
| 16 | t-Bu | Cl | S | Me<br>CH | CH | – | Cl<br>4-$CF_3$C=CH | |
| 17 | t-Bu | Cl | S | Me<br>CH | CH | – | 4-$Cl_2C$=CH | |
| 18 | t-Bu | Cl | S | Me<br>CH | CH | – | 4-$CF_3OCF$=CF | |
| 19 | t-Bu | Cl | S | $CH_2$ | CH | – | Cl<br>4-PhC=CH | |
| 20 | t-Bu | Cl | S | $CH_2$ | CH | – | Cl<br>4-(p-ClPh)C=CH | |
| 21 | t-Bu | Cl | S | $CH_2$ | CH | 3-Cl | 4-$CF_3OCFHCF_2O$ | 76 |
| 22 | t-Bu | Cl | S | $CH_2$ | CH | 3,5-$Cl_2$ | 4-$CF_3OCFHCF_2O$ | 98 |
| 23 | t-Bu | Cl | S | $CH_2$ | CH | 3-F | " | 97 |
| 24 | t-Bu | Cl | S | $CH_2$ | CH | 2-F | " | 74 |
| 25 | t-Bu | Cl | S | $CH_2CH_2$ | CH | – | " | |
| 26 | t-Bu | Cl | O | " | CH | – | " | |
| 27 | t-Bu | Cl | NH | $CH_2$ | CH | – | " | |
| 28 | t-Bu | Cl | S | 2-$CH_2$ | N | – | 5-$CF_3OCFHCF_2O$ | |
| 29 | t-Bu | Cl | S | $CH_2$ | CH | – | $CCl_2$<br>/ \\<br>4-$(CH_3)_2$C---CH | 140 |
| 30 | t-Bu | Cl | S | $CH_2$ | CH | – | $CCl_2$<br>/ \\<br>4-$CH_3$CH--CH | |
| 31 | t-Bu | Cl | S | $CH_2$ | CH | – | $CH_2$<br>/ \\<br>4-$Cl_2$C---C($CH_3$)$CH_2O$ | |
| 32 | t-Bu | Cl | S | $CH_2$ | CH | – | CH<br>/ \\<br>4-$Cl_2$C---C($CH_3$)CO | 161 |

| | | | | | | | |
|---|---|---|---|---|---|---|---|
| 33 | 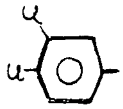 | Cl | O | $CH_2$ | CH | – | 4-$CF_3OCFHCF_2O$ |
| 34 | 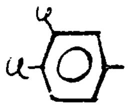 | Cl | S | $CH_2$ | CH | – | " |
| 35 | 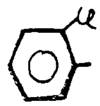 | Cl | O | $CH_2$ | CH | – | " |
| 36 | 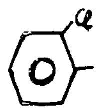 | Cl | S | " | " | – | " |
| 37 | 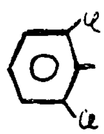 | Cl | O | " | " | – | " |
| 38 | 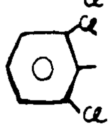 | Cl | S | " | " | – | " |
| 39 | 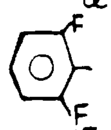 | Cl | O | " | " | – | " |
| 40 | 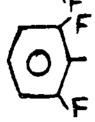 | Cl | S | " | " | – | " |
| $RC_1$ | t-Bu | Cl | O | Me<br>CH | CH | – | 4-$CH_3OCH_2CH_2O$ |
| $RC_2$ | t-Bu | Cl | S | Me<br>CH | CH | – | " |
| $RC_3$ | t-Bu | Cl | O | $CH_2$ | CH | – | " |
| $RC_4$ | t-Bu | Cl | S | $CH_2$ | CH | – | " |

Example 12

Determination of the insecticidal and acaricidal activity

a) Insecticidal activity against adults of Macrosiphum euphorbiae (M.E.; aphids)

Small potato plants grown in pot were infested with adult female aphids and, a few hours later, a suspension in water-acetone (acetone at 10% by volume) of the tested product was sprayed thereon.

24 hours after the treatment the percent mortality of the aphids was determined, as compared to the mortality rate of aphids infesting plants only treated with a solution of 10% of acetone in water.

b) Insecticidal activity against eggs and larvae of Leptinotarsa decemlineata (L.D.; coleoptera)

Potato leaves onto which living eggs of the species coleopter had been laid during the preceding 24 hours, were dipped in a water-acetone solution (at 10% by volume of acetone) of the tested product. The leaves were then placed on humidified filter paper.

6 days after the treatment the percentage of not opened eggs and of dead larvae vs. the percentage of eggs and larvae infesting leaves treated with only water-acetone solution (10%) was determined.

c) Acaricidal activity against Tetranychus urticae (T.U.; mites)
Adult mites
Small disks obtained from bean leaves were infested with adult mites and a water-acetone solution (acetone 10% by volume) of the tested product was then sprayed thereon. The percent mortality was determined after 48 hours of treatment, as compared to that of mites infesting disks onto which only an aqueous acetone-solution at 10% of acetone had been sprayed.
Eggs
Disks obtained from bean leaves were infested with eggs of the mite, and thereafter the disks were treated by being sprayed with a water-acetone solution of the tested product.
The percentage of not opened eggs was evaluated 7 days after the treatment, as compared to the percentage of eggs only treated with the water-acetone mixture.
Larvae
Small disks obtained form bean leaves were infested with eggs of the mite. 24 hours after the complete opening of the eggs, the juvenile forms of the mite were treated by being sprayed with a water-acetone solution of the tested product.
The percent mortality of the larvae was determined 72 hours after the treatment, as compared to that of larvae infesting disks onto which only a water-acetone solution had been sprayed.

By using the above procedures, the compounds according to the invention were tested in order to determine their insecticidal and acaricidal activity.

The results of said determinations are reported in Table 2. Said results are expressed as the percent mortality of the insects and mites treated with the tested compounds, at the doses indicated.

Table 2

| Compound | M.E. 100 ppm | L.D. 1000 ppm | T.U. adults 10 ppm | T.U. eggs 10 ppm |
|---|---|---|---|---|
| 1 | 100 | 100 | 100 | 100 |
| 2 | 100 | 100 | 100 | 100 |
| 3 | 100 | 100 | 100 | 100 |
| 4 | 100 | 100 | 100 | 100 |
| 5 | 100 | n.t. | 100 | 100 |
| 6 | 100 | n.t. | 100 | 100 |
| 7 | 100 | 100 | 100 | 100 |
| 8 | 100 | 100 | 100 | 100 |
| 9 | 96 | 100 | 100 | 100 |
| 10 | 100 | 100 | 100 | 100 |
| 11 | 100 | 100 | 100 | 100 |
| 12 | 100 | 100 | 100 | 100 |
| 13 | 100 | 100 | 100 | 100 |
| 14 | 100 | 100 | 100 | 100 |
| 15 | 90 | 100 | 100 | 100 |
| 16 | 100 | 100 | 100 | 100 |
| 17 | 100 | 100 | 100 | 100 |
| 18 | 100 | 100 | 100 | 100 |
| 21 | 100 | 100 | 100 | 100 |
| 22 | 100 | 100 | 100 | 95 |
| 23 | 100 | 100 | 100 | 100 |
| 30 | 100 | 100 | 100 | 100 |

nt = not tested

In Table 3 are reported the data of acaricidal activity (adults, eggs, larvae) of compounds 1 to 4 in comparison with the data of acaricidal activity of the reference compounds $RC_1$ to $RC_4$ of EP-A-199281.

Table 3

| Compound | T.U. adults | T.U. eggs | T.U. larvae |
|---|---|---|---|
| | 10 ppm | 10 ppm | 10 ppm |
| | 1 ppm | 1 ppm | 1 ppm |
| | 0.1ppm | 0.1ppm | 0.1ppm |
| 1 | 100<br>42<br>nt | 100<br>54<br>nt | 98<br>90<br>41 |
| $RC_1$ | 18<br>7<br>nt | 22<br>10<br>nt | 18<br>9<br>nt |
| 2 | 100<br>100<br>90 | 100<br>100<br>95 | 100<br>100<br>100 |
| $RC_2$ | 100<br>100<br>13 | 100<br>98<br>6 | 100<br>98<br>70 |
| 3 | 100<br>71<br>nt | 100<br>90<br>nt | 100<br>85<br>53 |
| $RC_3$ | 7<br>0<br>nt | 77<br>24<br>nt | 33<br>0<br>nt |
| 4 | 100<br>90<br>70 | 100<br>100<br>65 | 100<br>100<br>100 |
| $RC_4$ | 80<br>42<br>4 | 100<br>90<br>55 | 100<br>96<br>32 |

## Claims

**Claims for the following Contracting States : AT, BE, CH, DE, FR, GB LI, NL**

**1.** Pyridazinones of general formula (I):

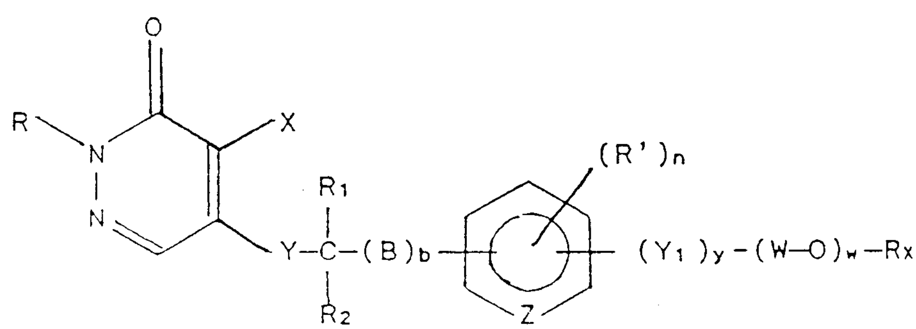

(I)

wherein:

R represents a linear or branched ($C_2$-$C_6$) alkyl group; or a phenyl group, optionally substituted by halogen atoms and/or ($C_{1-6}$) alkyl, ($C_{1-6}$) haloalkyl,

($C_{1-6}$) alkoxy and/or ($C_{1-6}$) haloalkoxy radicals;

X represents a halogen atom;

Y represents O, S or NH;

B represents:

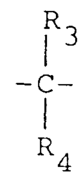

b is O or 1;

$R_1$, $R_2$, $R_3$ and $R_4$ independently represent H or a linear or branched ($C_1$-$C_4$) alkyl or ($C_{1-6}$) haloalkyl group;

Z represents CH or N;

$Y_1$ represents O, S, CO or

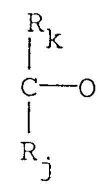

wherein:

$R_k$ and $R_j$ independently are H or a linear or branched ($C_1$-$C_4$) alkyl or ($C_{1-6}$) haloalkyl group;

W is a linear or branched ($C_2$-$C_6$) haloalkylene group;

y and w independently are O or 1;

$R_x$ for w = O, represents a linear or branched ($C_2$-$C_6$) haloalkenyl group optionally having a ($C_1$-$C_{3)}$ haloalkoxy or phenyl group as substituent, said phenyl group in turn being optionally substituted by one or more halogen atoms, ($C_1$-$C_6$) alkyl, ($C_{1-6}$) haloalkyl, ($C_{1-6}$) alkoxy and/or ($C_{1-6}$) haloalkoxy radicals; or represents a ($C_3$-$C_6$) halocycloalkyl or ($C_4$-$C_7$) halocycloalkylalkyl radical, which radicals may have one or more substituents selected from ($C_1$-$C_4$) alkyl or ($C_{1-6}$) haloalkyl groups; and, for w = 1, represents a linear or branched ($C_1$-$C_6$) haloalkyl radical;

R' represents a halogen atom; and

n is O, 1 or 2.

**2.** Compounds according to claim 1, having the general formula:

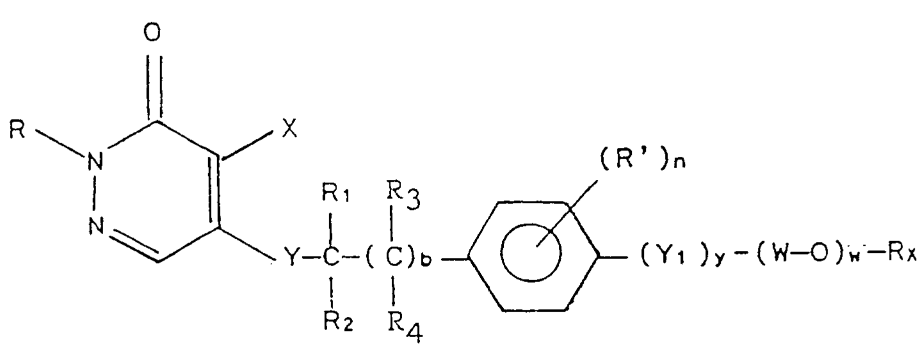

(Ia)

wherein:

R represents a ($C_2$-$C_6$) alkyl radical;

Y represents 0 or S;
the remaining groups being defined as in claim 1.

**3.** Compounds according to any one of claims 1 and 2, wherein R is butyl or optionally substituted phenyl.

**4.** Compounds according to claim 3, wherein R is tert.-butyl or chloro and/or fluoro-substituted phenyl.

**5.** Compounds according to any one of claims 1 to 4, wherein X if F, Cl or Br.

**6.** Compounds according to claim 5, wherein X is Cl.

**7.** Compounds according to any one of claims 1 to 6, wherein $R_1$ is H or $C_{1-4}$-alkyl, $R_2$ is H and b is 0.

**8.** Compounds according to claim 7, wherein $R_1$ is methyl.

**9.** Compounds according to any one of claims 1 to 8, wherein the moiety $-(Y_1)_y-(W-O)_w-R_x$ contains at least one F and/or Cl atom.

**10.** Compounds according to claim 9, wherein the moiety $-(Y_1)_y-(W-O)_w-R_x$ is selected from 1,1,2-trifluoro-2-(partly or completely fluorinated $C_{1-4}$-alkoxy)-ethoxy, 2-chloro-2-(partly or completely fluorinated $C_{1-4}$-alkyl)-ethenyl, 2,2-dichloro-ethenyl, 1,2-difluoro-2-(partly or completely fluorinated $C_{1-4}$-alkoxy)-ethenyl, 2-chloro-2-(optionally substituted phenyl)-ethenyl, $\omega$ -(2,2,-dichloro-1-methyl-cyclopropyl)-$C_{1-4}$-alkoxy and (2,2-dichloro-1-methyl-cyclopropyl)-carbonyl.

**11.** Compounds according to claim 1, i.e.,
2-tert.-butyl-4-chloro-5-[4-(1,1,2-trifluoro-2-trifluoromethoxy-ethoxy)-α-methyl-benzyloxy]-3(2H)-pyridazinone,
2-tert.-butyl-4-chloro-5-[4-(1,1,2-trifluoro-2-trifluoromethoxy-ethoxy)-α-methyl-benzylthio]-3(2H)-pyridazinone,
2-tert.-butyl-4-chloro-5-[4-(1,1,2-trifluoro-2-trifluoromethoxy-ethoxy)-benzyloxy]-3(2H)-pyridazinone,
2-tert.-butyl-4-chloro-5-[4-(1,1,2-trifluoro-2-trifluoromethoxy-ethoxy)-benzylthio]-3(2H)-pyridazinone,
2-tert.-butyl-4-chloro-5-[4-(2-pentafluoroethoxy-1,1,2-trifluoroethoxy)-benzylthio]-3(2H)-pyridazinone,
2-tert.-butyl-4-chloro-5-[4-(2-pentafluoroethoxy-1,1,2-trifluoro-ethoxy)-α-methylbenzylthio]-3(2H)-pyridazinone,
2-tert.-butyl-4-chloro-5-[4-(2-chloro-3,3,3-trifluoropropenyl)-benzylthio]-3(2H)-pyridazinone,
2-tert.-butyl-4-chloro-5-[4-(2,2-dichloro-ethenyl)-benzylthio]-3(2H)-pyridazinone,
2-tert.-butyl-4-chloro-5-[4-(1,1,2-trifluoro-2-trifluoromethoxy-ethoxy-ethyl)-benzylthio]-3(2H)-pyridazinone,
2-tert.-butyl-4-chloro-5-[4-(1,2-difluoro-2-trifluoromethoxy-ethenyl)-benzylthio]-3(2H)-pyridazinone,
2-tert.-butyl-4-chloro-5-[4-(2-chloro-3,3,3-trifluoropropenyl)-α-methylbenzylthio]-3(2H)-pyridazinone,
2-tert.-butyl-4-chloro-5-[4-(2,2-dichloroethenyl)-α-methyl-benzylthio]-3(2H)-pyridazinone,
2-tert.-butyl-4-chloro-5[4-(1,2-difluoro-2-trifluoromethoxy-ethenyl)-α-methylbenzylthio]-3(2H)-pyridazinone,
2-tert.-butyl-4-chloro-5-[4-(2-chloro-2-phenylethenyl)-benzylthio]-3(2H)-pyridazinone,
2-tert.-butyl-4-chloro-5-[4-(2-chloro-(4-chlorophenylethenyl)-benzylthio]-3(2H)-pyridazinone,
2-tert.-butyl-4-chloro-5-[4-(2,2-dichloro-1-methylcyclopropyl-methoxy)-benzylthio]-3(2H)-pyridazinone,
2-tert.-butyl-4-chloro-5-[4-(2,2-dichloro-1-methylcyclopropyl-carbonyl)-benzylthio]-3(2H)-pyridazinone,
2-(3,4-dichlorophenyl)-4-chloro-5-[4-(1,1,2-trifluoro-2-trifluoromethoxy-ethoxy)-benzyloxy]-3(2H)-pyridazinone and
2-(3,4-dichlorophenyl)-4-chloro-5-[4-(1,1,2-trifluoro-2-trifluoromethoxy-ethoxy)-benzylthio]-3(2H)-pyridazinone.

**12.** Process for preparing the compounds of claim 1, characterized in that a pyridazinone of formula (II):

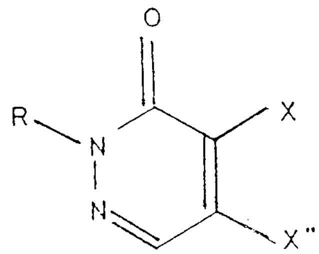

(II)

is reacted with a compound of formula (III):

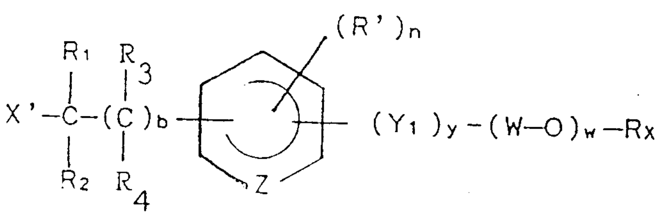

(III)

in which formulae the symbols R, X, $R_1$, $R_2$, $R_3$, $R_4$, Z, $Y_1$, W, $R_x$, R', b, y, w, and n have the meanings indicated in claim 1 and X' and X" represent Cl, Br, I or YH, Y being defined as in claim 1, with the proviso that one of X' and X" represents YH whereas the other one represents Cl, Br, or I;
in an organic solvent, in the presence of an inorganic or organic base and at a temperature of from room temperature to the reflux temperature of the reaction mixture.

**13.** Method for controlling infestations by harmful mites and/or insects in agricultural cultivations, horticultural cultivations and/or other areas, comprising the application of at least one pyridazinone according to any one of claims 1 to 11 onto said cultivation and/or area.

**14.** Compositions for controlling infestations by harmful mites and/or insects, comprising at least one pyridazinone according to any one of claims 1 to 11, one or more solid or liquid carriers and, optionally, further usual additives and/or active ingredients and/or fertilizers.

**Claims for the following Contracting State : ES**

**1.** A process for preparing pyridazinones of general formula (I):

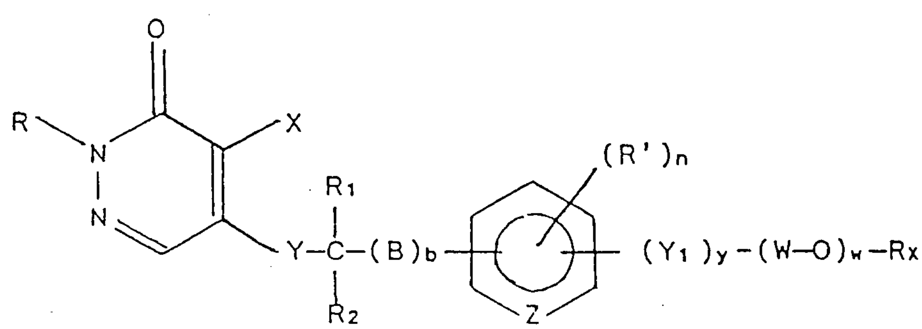

(I)

wherein:

| | |
|---|---|
| R | represents a linear or branched ($C_2$-$C_6$) alkyl group; or a phenyl group, optionally substituted by halogen atoms and/or ($C_1$-$C_6$) alkyl, ($C_1$-$C_6$) haloalkyl, ($C_1$-$C_6$) alkoxy and/or ($C_1$-$C_6$) haloalkoxy radicals; |
| X | represents a halogen atom; |

Y represents O, S or NH;

B represents:

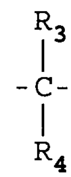

b is 0 or 1;

$R_1$, $R_2$, $R_3$ and $R_4$ independently represent H or a linear or branched ($C_1$-$C_4$) alkyl or ($C_1$-$C_6$) haloalkyl group;

Z represents CH or N;

$Y_1$ represents O, S, CO or

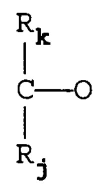

wherein $R_k$ and $R_j$ independently are H or a linear or branched ($C_1$-$C_4$) alkyl or ($C_1$-$C_6$) haloalkyl group;

W is a linear or branched ($C_2$-$C_6$) haloalkylene group;

y and w independently are 0 or 1;

$R_x$ for w = 0, represents a linear or branched ($C_2$-$C_6$) haloalkenyl group optionally having a ($C_1$-$C_3$) haloalkoxy or phenyl group as substituent, said phenyl group in turn being optionally substituted by one or more halogen atoms, ($C_1$-$C_6$) alkyl, ($C_1$-$C_6$) haloalkyl, ($C_1$-$C_6$) alkoxy and/or ($C_1$-$C_6$) haloalkoxy radicals; or represents a ($C_3$-$C_6$) halocycloalkyl or ($C_4$-$C_7$) halocycloalkylalkyl radical, which radicals may have one or more substituents selected from ($C_1$-$C_4$) alkyl or ($C_1$-$C_6$) haloalkyl groups; and, for w = 1, represents a linear or branched ($C_1$-$C_6$) haloalkyl radical;

R' represents a halogen atom; and

n is 0, 1 or 2,

characterized in that a pyridazinone of formula (II):

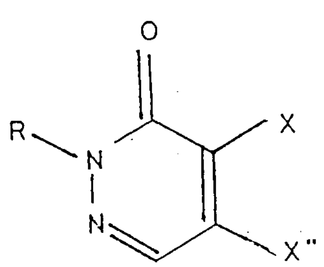

(II)

is reacted with a compound of formula (III):

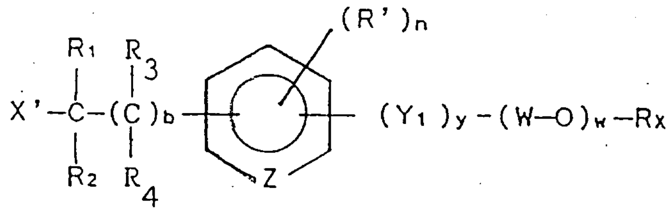

(III)

in which formulae the symbols R, X, $R_1$, $R_2$, $R_3$, $R_4$, Z, $Y_1$, W, $R_x$, R', b, y, w, and n have the above meanings and X' and X'' represent Cl, Br, I or YH, Y being defined as above, with the proviso that one of X' and X'' represents YH whereas the other one represents Cl, Br, or I;
in an organic solvent, in the presence of an inorganic or organic base and at a temperature of from room temperature to the reflux temperature of the reaction mixture.

**2.** A process according to claim 1, wherein compounds having the general formula:

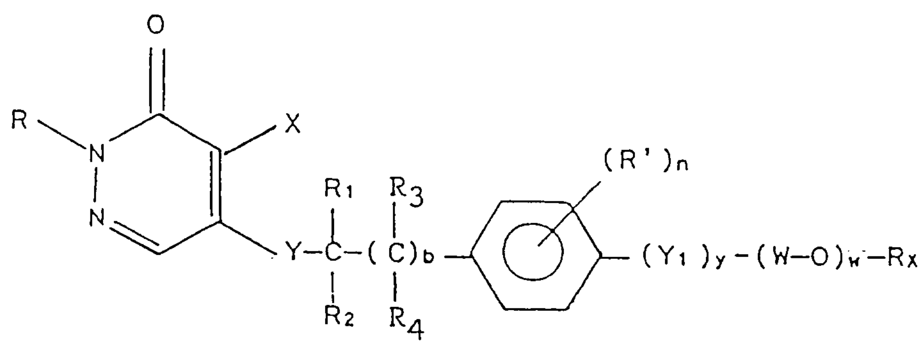

(Ia)

wherein:
R represents a ($C_2$-$C_6$) alkyl radical;
Y represents O or S;
the remaining groups being defined as in claim 1, are prepared.

**3.** A process according to any one of claims 1 and 2, wherein R is butyl or optionally substituted phenyl.

**4.** A process according to claim 3, wherein R is tert.-butyl or chloro and/or fluoro-substituted phenyl.

**5.** A process according to any one of claims 1 to 4, wherein X if F, Cl or Br.

**6.** A process according to claim 5, wherein X is Cl.

**7.** A process according to any one of claims 1 to 6, wherein $R_1$ is H or $C_{1-4}$-alkyl, $R_2$ is H and b is 0.

**8.** A process according to claim 7, wherein $R_1$ is methyl.

**9.** A process according to any one of claims 1 to 8, wherein the moiety -$(Y_1)_y$-$(W\text{-}O)_w$-$R_x$ contains at least one F and/or Cl atom.

**10.** A process according to claim 9, wherein the moiety -$(Y_1)_y$-$(W\text{-}O)_w$-$R_x$ is selected from 1,1,2-trifluoro-2-(partly or completely fluorinated $C_{1-4}$-alkoxy)-ethoxy, 2-chloro-2-(partly or completely fluorinated $C_{1-4}$-alkyl)-ethenyl, 2,2-dichloro-ethenyl, 1,2-difluoro-2-(partly or completely fluorinated $C_{1-4}$-alkoxy)-ethenyl, 2-chloro-2-(optionally substituted phenyl)-ethenyl, ω-(2,2,-dichloro-1-methyl-cyclopropyl)-$C_{1-4}$-alkoxy and (2,2-dichloro-1-methyl-cyclopropyl)-carbonyl.

**11.** A process according to claim 1, wherein the following compounds are prepared:
2-tert.-butyl-4-chloro-5-[4-(1,1,2-trifluoro-2-trifluoromethoxy-ethoxy)-α-methyl-benzyloxy]-3(2H)-pyridazinone,
2-tert.-butyl-4-chloro-5-[4-(1,1,2-trifluoro-2-trifluoromethoxy-ethoxy)-α-methyl-benzylthio]-3(2H)-pyridazinone,
2-tert.-butyl-4-chloro-5-[4-(1,1,2-trifluoro-2-trifluoromethoxy-ethoxy)-benzyloxy]-3(2H)-pyridazinone,
2-tert.-butyl-4-chloro-5-[4-(1,1,2-trifluoro-2-trifluoromethoxy-ethoxy)-benzylthio]-3(2H)-pyridazinone,
2-tert.-butyl-4-chloro-5-[4-(2-pentafluoroethoxy-1,1,2-trifluoroethoxy)-benzylthio]-3(2H)-pyridazinone,
2-tert.-butyl-4-chloro-5-[4-(2-pentafluoroethoxy-1,1,2-trifluoro-ethoxy)-α-methylbenzylthio]-3(2H)-pyridazinone,
2-tert.-butyl-4-chloro-5-[4-(2-chloro-3,3,3-trifluoropropenyl)-benzylthio]-3(2H)-pyridazinone,
2-tert.-butyl-4-chloro-5-[4-(2,2-dichloro-ethenyl)-benzylthio]-3(2H)-pyridazinone,
2-tert.-butyl-4-chloro-5-[4-(1,1,2-trifluoro-2-trifluoromethoxy-ethoxy-ethyl)-benzylthio]-3(2H)-pyridazinone,
2-tert.-butyl-4-chloro-5-[4-(1,2-difluoro-2-trifluoromethoxy-ethenyl)-benzylthio]-3(2H)-pyridazinone,
2-tert.-butyl-4-chloro-5-[4-(2-chloro-3,3,3-trifluoropropenyl)-α-methylbenzylthio]-3(2H)-pyridazinone,
2-tert.-butyl-4-chloro-5-[4-(2,2-dichloroethenyl)-α-methyl-benzylthio]-3(2H)-pyridazinone,
2-tert.-butyl-4-chloro-5[4-(1,2-difluoro-2-trifluoromethoxy-ethenyl)-α-methylbenzylthio]-3(2H)-pyridazinone,
2-tert.-butyl-4-chloro-5-[4-(2-chloro-2-phenylethenyl)-benzylthio]-3(2H)-pyridazinone,
2-tert.-butyl-4-chloro-5-[4-(2-chloro-(4-chlorophenylethenyl)-benzylthio]-3(2H)-pyridazinone,
2-tert.-butyl-4-chloro-5-[4-(2,2-dichloro-1-methylcyclopropyl-methoxy)-benzylthio]-3(2H)-pyridazinone,
2-tert.-butyl-4-chloro-5-[4-(2,2-dichloro-1-methylcyclopropyl-carbonyl)-benzylthio]-3(2H)-pyridazinone,
2-(3,4-dichlorophenyl)-4-chloro-5-[4-(1,1,2-trifluoro2-trifluoromethoxy-ethoxy)-benzyloxy]-3(2H)-pyridazinone and
2-(3,4-dichlorophenyl)-4-chloro-5-[4-(1,1,2-trifluoro-2-trifluoromethoxy-ethoxy)-benzylthio]-3(2H)-pyridazinone.

**12.** Method for controlling infestations by harmful mites and/or insects in agricultural cultivations, horticultural cultivations and/or other areas, comprising the application of at least one pyridazinone according to any one of claims 1 to 11 onto said cultivation and/or area.

**13.** Compositions for controlling infestations by harmful mites and/or insects, comprising at least one pyridazinone according to any one of claims 1 to 11, one or more solid or liquid carriers and, optionally, further usual additives and/or active ingredients and/or fertilizers.

**Patentansprüche**

**Patentansprüche für folgende Vertragsstaaten : AT, BE, CH, DE, FR, GB, LI, NL**

**1.** Pyridazinone der allgemeinen Formel (I):

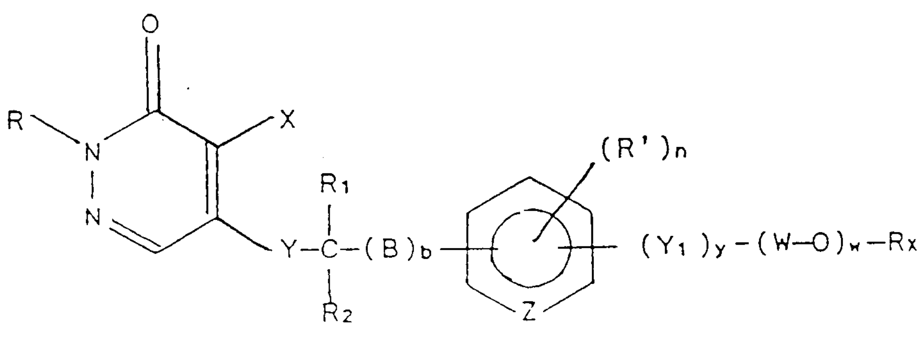

(I)

worin:

| | |
|---|---|
| R | eine lineare oder verzweigte ($C_2$-$C_6$) Alkylgruppe; oder eine Phenylgruppe, gegebenenfalls durch Halogenatome und/oder ($C_{1-6}$) Alkyl-, ($C_{1-6}$) Halogenalkyl-, ($c_{1-6}$) Alkoxy- und/oder ($C_{1-6}$) Halogenalkoxy-Reste substituiert, darstellt; |
| X | für ein Halogenatom steht; |
| Y | O, S oder NH bedeutet; |
| B | |

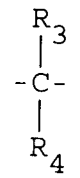

darstellt;

- b — 0 oder 1 ist;
- $R_1$, $R_2$, $R_3$ und $R_4$ — unabhängig für H oder eine lineare oder verzweigte ($C_1$-$C_4$) Alkyl- oder ($C_{1-6}$) Halogenalkyl-Gruppe stehen;
- Z — CH oder N darstellt;
- $Y_1$ — für O, S, CO oder

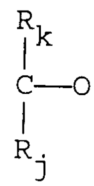

steht;
worin:
$R_k$ und $R_j$ unabhängig H oder eine lineare oder verzweigte ($C_1$-$C_4$) Alkyl- oder ($C_{1-6}$) Halogenalkyl-Gruppe repräsentieren;

- W — eine lineare oder verzweigte ($C_2$-$C_6$) Halogenalkylen-Gruppe ist;
- y — und w unabhängig 0 oder 1 sind;
- $R_x$ — für w = 0 eine lineare oder verzweigte ($C_2$-$C_6$) Halogenalkenylgruppe, die gegebenenfalls eine ($C_1$-$C_3$) Halogenalkoxy- oder Phenyl-Gruppe als Substituent aufweist, wobei die Phenylgruppe ihrerseits gegebenenfalls durch ein(en) oder mehrere Halogenatome, ($C_1$-$C_6$) Alkyl-, ($C_{1-6}$) Halogenalkyl-, ($C_{1-6}$) Alkoxy- und/oder ($C_{1-6}$) Halogenalkoxy-Reste substituiert ist, darstellt; oder einen ($C_3$-$C_6$) Halogencycloalkyl- oder ($C_4$-$C_7$) Halogencycloalkylalkyl-Rest darstellt, wobei diese Reste einen oder mehrere Substituenten, ausgewählt aus ($C_1$-$C_4$) Alkyl- oder ($C_{1-6}$)-Halogenalkyl-Gruppen, aufweisen können; und für w = 1 einen linearen oder verzweigten ($C_1$-$C_6$) Halogenalkyl-Rest darstellt;
- R' — für ein Halogenatom steht; und
- n — 0, 1 oder 2 ist.

**2.** Verbindungen nach Anspruch 1 mit der allgemeinen Formel:

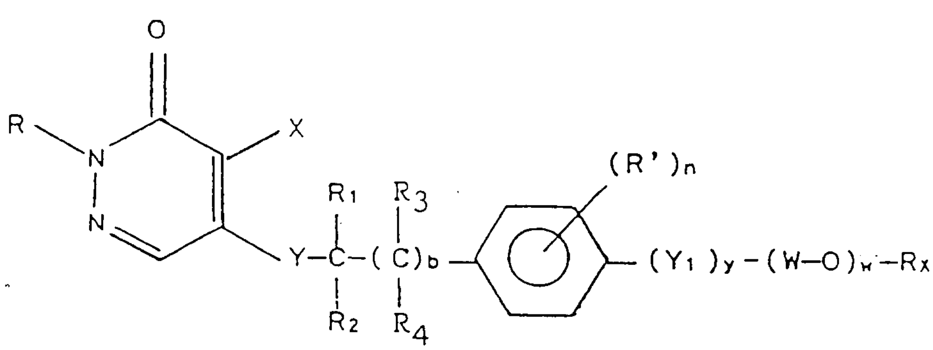

(Ia)

worin:

- R — für einen ($C_2$-$C_6$) Alkyl-Rest steht;
- Y — O oder S bedeutet;

wobei die verbleibenden Gruppen wie in Anspruch 1 definiert sind.

**3.** Verbindungen nach irgendeinem der Ansprüche 1 und 2, in denen R Butyl oder gegebenenfalls substituiertes Phenyl ist.

**4.** Verbindungen nach Anspruch 3, in denen R tert-Butyl oder Chlor- und/oder Fluor-substituiertes Phenyl ist.

**5.** Verbindungen nach irgendeinem der Ansprüche 1 bis 4, in denen X für F, Cl oder Br steht.

**6.** Verbindungen nach Anspruch 5, in denen X für Cl steht.

**7.** Verbindungen nach irgendeinem der Ansprüche 1 bis 6, in denen $R_1$ H oder $C_{1-4}$-Alkyl ist, $R_2$ H bedeutet und b 0 ist.

**8.** Verbindungen nach Anspruch 7, in denen $R_1$ Methyl ist.

**9.** Verbindungen nach irgendeinem der Ansprüche 1 bis 8, in denen die Einheit $-(Y_1)_y-(W-O)_w-R_x$ mindestens ein F- und/oder Cl-Atom enthält.

**10.** Verbindungen nach Anspruch 9, in denen die Einheit $-(Y_1)_y-(W-O)_w-R_x$ ausgewählt ist aus 1,1,2-Trifluor-2-(teilweise oder vollständig fluoriertes $C_{1-4}$-alkoxy)-ethoxy, 2-Chlor-2-(teilweise oder vollständig fluoriertes $C_{1-4}$-alkyl)-ethenyl, 2,2-Dichlorethenyl, 1,2-Difluor-2-(teilweise oder vollständig fluoriertes $C_{1-4}$-alkoxy)-ethenyl, 2-Chlor-2-(gegebenenfalls substituiertes phenyl)-ethenyl, $\omega$-(2,2-Dichlor-1-methylcyclopropyl)-$C_{1-4}$-alkoxy und (2,2-Dichlor-1-methyl-cyclopropyl)-carbonyl.

**11.** Verbindungen nach Anspruch 1, nämlich 2-tert.-Butyl-4-chlor-5-[4-(1,1,2-trifluor-2-trifluormethoxyethoxy)-$\alpha$-methyl-benzyloxy]-3(2H)-pyridazinon, 2-tert.-Butyl-4-chlor-5-[4-(1,1,2-trifluor-2-trifluormethoxyethoxy)-$\alpha$-methyl-benzylthio]-3(2H)-pyridazinon, 2-tert.-Butyl-4-chlor-5-[4-(1,1,2-trifluor-2-trifluormethoxyethoxy)-benzyloxy]-3(2H)-pyridazinon, 2-tert.-Butyl-4-chlor-5-[4-(1,1,2-trifluor-2-trifluormethoxyethoxy)-benzylthio]-3(2H)-pyridazinon, 2-tert.-Butyl-4-chlor-5-[4-(2-pentafluorethoxy-1,1,2-trifluorethoxy)-benzylthio]-3(2H)-pyridazinon, 2-tert.-Butyl-4-chlor-5-[4-(2-pentafluorethoxy-1,1,2-trifluorethoxy)-$\alpha$-methylbenzylthio]-3(2H)-pyridazinon, 2-tert.-Butyl-4-chlor-5-[4-(2-chlor-3,3,3-trifluorpropenyl)-benzylthio]-3(2H)-pyridazinon, 2-tert.-Butyl-4-chlor-5-[4-(2,2-dichlorethenyl)-benzylthio]-3(2H)-pyridazinon, 2-tert.-Butyl-4-chlor-5-[4-(1,1,2-trifluor-2-trifluormethoxyethoxyethyl)-benzylthio]-3(2H)-pyridazinon, 2-tert.-Butyl-4-chlor-5-[4-(1,2-difluor2-trifluormethoxyethenyl)-benzylthio]-3(2H)-pyridazinon, 2-tert.-Butyl-4-chlor-5-[4-(2-chlor-3,3,3-trifluorpropenyl)-$\alpha$-methylbenzylthio]-3(2H)-pyridazinon, 2-tert.-Butyl-4-chlor-5-[4-(2,2-dichlorethenyl)-$\alpha$-methyl-benzylthio]-3(2H)-pyridazinon, 2-tert.-Butyl-4-chlor-5-[4-(1,2-difluor-2-trifluormethoxyethenyl)-$\alpha$-methylbenzylthio]-3(2H)-pyridazinon, 2-tert.-Butyl-4-chlor-5-[4-(2-chlor-2-phenylethenyl)-benzylthio]-3-(2H)-pyridazinon, 2-tert.-Butyl-4-chlor-5-[4-(2-chlor(4-chlorphenylethenyl)-benzylthio]-3(2H)-pyridazinon, 2-tert.-Butyl-4-chlor-5-[4-(2,2-dichlor-1-methyl-cyclopropylmethoxy)-benzylthio]-3(2H)-pyridazinon, 2-tert.-Butyl-4-chlor5-[4-(1,2-dichlor-1-methylcyclopropylcarbonyl)-benzylthio]-3(2H)-pyridazinon, 2-(3,4-Dichlorphenyl)-4-chlor-5-[4-(1,1,2-trifluor-2-trifluor-methoxyethoxy)-benzyloxy]-3(2H)-pyridazinon und 2-(3,4-Dichlorphenyl)-4-chlor-5-[4-(1,1,2-trifluor-2-trifluormethoxyethoxy)-benzylthio]-3(2H)-pyridazinon.

**12.** Verfahren zur Herstellung der Verbindungen nach Anspruch 1, dadurch gekennzeichnet, daß ein Pyridazinon der Formel (II):

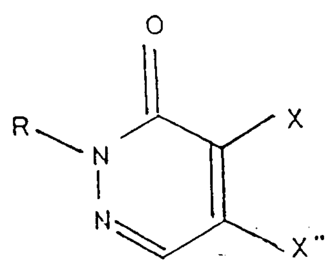

(II)

mit einer Verbindung der Formel (III):

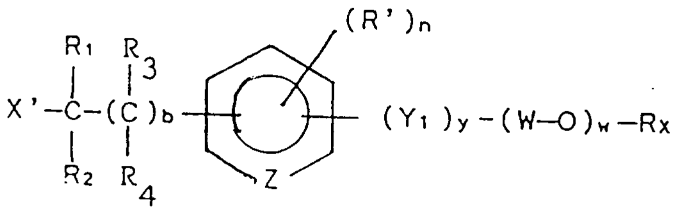

(III)

wobei in diesen Formeln die Symbole R, X, $R_1$, $R_2$, $R_3$, $R_4$, Z, $Y_1$, W, $R_x$, R', b, y, w und n die in Anspruch 1 angegebenen Bedeutungen aufweisen und X' und X" für Cl, Br, I oder YH, wobei Y wie in Anspruch 1 definiert ist, stehen, mit der Maßgabe, daß eines von X' und X" für YH steht, während das andere Cl, Br oder I darstellt;
in einem organischen Lösungsmittel in Anwesenheit einer anorganischen oder organischen Base und bei einer Temperatur von Raumtemperatur bis zur Rückflußtemperatur der Reaktionsmischung umgesetzt wird.

**13.** Verfahren zur Kontrolle des Befalls durch schädliche Milben und/oder Insekten im landwirtschaftlichen Anbau, in Gartenbau-Kulturen und/oder anderen Bereichen, umfassend die Anwendung mindestens eines Pyridazinons nach irgendeinem der Ansprüche 1 bis 11 auf den Anbau und/oder den Bereich.

**14.** Zusammensetzungen zur Kontrolle des Befalls durch schädliche Milben und/oder Insekten, umfassend mindestens ein Pyridazinon gemäß irgendeinem der Ansprüche 1 bis 11, einen oder mehrere feste oder flüssige Trägerstoffe und gegebenenfalls weitere übliche Additive und/oder aktive Bestandteile und/oder Düngemittel.

**Patentansprüche für folgenden Vertragsstaat : ES**

**1.** Verfahren zur Herstellung von Pyridazinonen der allgemeinen Formel (I):

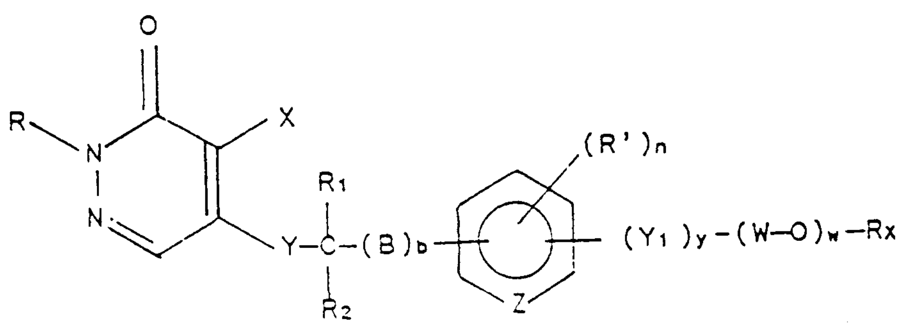

(I)

worin:

| | |
|---|---|
| R | eine lineare oder verzweigte ($C_2$-$C_6$) Alkylgruppe; oder eine Phenylgruppe, gegebenenfalls durch Halogenatome und/oder ($C_{1-6}$) Alkyl-, ($C_{1-6}$) Halogenalkyl-, ($C_{1-6}$) Alkoxy- und/oder ($C_{1-6}$) Halogenalkoxy-Reste substituiert, darstellt; |
| X | für ein Halogenatom steht; |
| Y | O, S oder NH bedeutet; |
| B | |

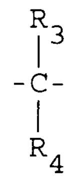

darstellt;

b 0 oder 1 ist;

$R_1$, $R_2$, $R_3$ und $R_4$ unabhängig für H oder eine lineare oder verzweigte ($C_1$-$C_4$) Alkyl- oder ($C_{1-6}$) Halogenalkyl-Gruppe stehen;

Z CH oder N darstellt;

$Y_1$ für O, S, CO oder

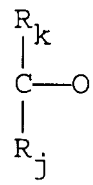

steht;

worin:

$R_k$ und $R_j$ unabhängig H oder eine lineare oder verzweigte ($C_1$-$C_4$) Alkyl- oder ($C_{1-6}$) Halogenalkyl-Gruppe repräsentieren;

W eine lineare oder verzweigte ($C_2$-$C_6$) Halogenalkylen-Gruppe ist;

y und w unabhängig 0 oder 1 sind;

$R_x$ für w = 0 eine lineare oder verzweigte ($C_2$-$C_6$) Halogenalkenylgruppe, die gegebenenfalls eine ($C_1$-$C_3$) Halogenalkoxy- oder Phenyl-Gruppe als Substituent aufweist, wobei die Phenylgruppe ihrerseits gegebenenfalls durch ein(en) oder mehrere Halogenatome, ($C_1$-$C_6$) Alkyl-, ($C_{1-6}$) Halogenalkyl-, ($C_{1-6}$) Alkoxy- und/oder ($C_{1-6}$) Halogenalkoxy-Reste substituiert ist, darstellt; oder einen ($C_3$-$C_6$) Halogencycloalkyl- oder ($C_4$-$C_7$) Halogencycloalkylalkyl-Rest darstellt, wobei diese Reste einen oder mehrere Substituenten, ausgewählt aus ($C_1$-$C_4$) Alkyl- oder ($C_{1-6}$)-Halogenalkyl-Gruppen, aufweisen können; und für w = 1 einen linearen oder verzweigten ($C_1$-$C_6$) Halogenalkyl-Rest darstellt;

R' für ein Halogenatom steht; und

n 0, 1 oder 2 ist,

dadurch gekennzeichnet, daß ein Pyridazinon der Formel (II):

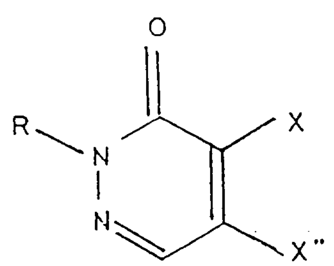

(II)

mit einer Verbindung der Formel (III):

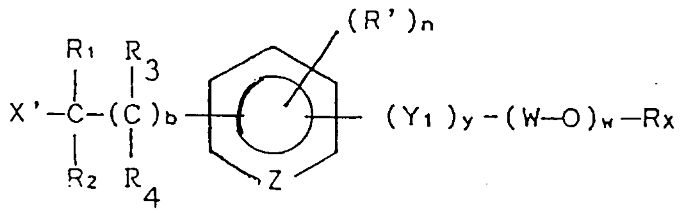

(III)

wobei in diesen Formeln die Symbole R, X, $R_1$, $R_2$, $R_3$, $R_4$, Z, $Y_1$, W, $R_x$, R', b, y, w und n die obigen Bedeutungen aufweisen und X' und X" für Cl, Br, I oder YH, wobei y wie oben definiert ist, stehen, mit der Maßgabe, daß eines von X' und X" für YH steht während das andere Cl, Br oder I darstellt; in einem organischen Lösungsmittel in Anwesenheit einer anorganischen oder organischen Base und bei einer Temperatur von Raumtemperatur bis zur Rückflußtemperatur der Reaktionsmischung umgesetzt wird.

**2.** Verfahren nach Anspruch 1, in welchem Verbindungen der allgemeinen Formel:

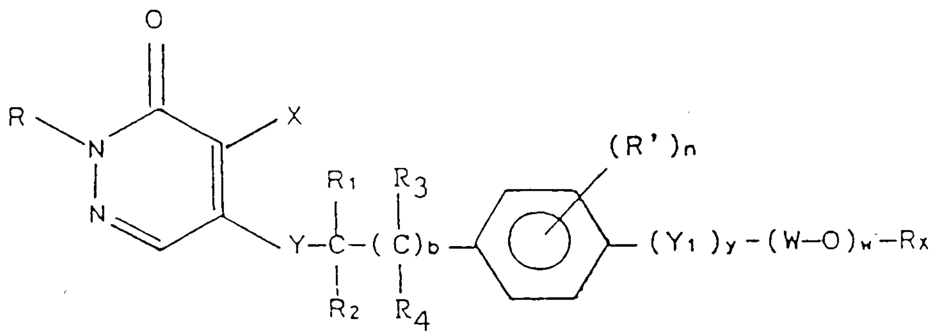

(Ia)

worin:

R für einen ($C_2$-$C_6$) Alkyl-Rest steht;

y O oder S bedeutet;

wobei die verbleibenden Gruppen wie in Anspruch 1 definiert sind, hergestellt werden.

**3.** Verfahren nach irgendeinem der Ansprüche 1 und 2, in welchem R Butyl oder gegebenenfalls substituiertes Phenyl ist.

**4.** Verfahren nach Anspruch 3, in welchem R für tert.-Butyl oder Chlor- und/oder Fluor-substituiertes Phenyl steht.

**5.** Verfahren nach irgendeinem der Ansprüche 1 bis 4, in welchem X für F, Cl oder Br steht.

**6.** Verfahren nach Anspruch 5, in welchem X für Cl steht.

**7.** Verfahren nach irgendeinem der Ansprüche 1 bis 6, in welchem $R_1$ für H oder $C_{1-4}$-Alkyl steht, $R_2$ H bedeutet und b 0 ist.

**8.** Verfahren nach Anspruch 7, in welchem $R_1$ Methyl ist.

**9.** Verfahren nach irgendeinem der Ansprüche 1 bis 8, in welchem die Einheit -$(Y_1)_y$-$(W\text{-}O)_w$-$R_x$ mindestens ein F- und/oder Cl-Atom enthält.

**10.** Verfahren nach Anspruch 9, in welchem die Einheit -$(Y_1)_y$-$(W\text{-}O)_w$-$R_x$ ausgewählt wird aus 1,1,2-Trifluor-2-(teilweiseoder vollständig fluoriertes $C_{1-4}$-alkoxy)-ethoxy, 2-Chlor-2-(teilweise oder vollständig fluoriertes $C_{1-4}$-alkyl)-ethenyl, 2,2-Dichlorethenyl, 1,2-Difluor-2-(teilweise oder vollständig fluoriertes $C_{1-4}$-

alkoxy)-ethenyl, 2-Chlor-2-(gegebenenfalls substituiertes phenyl)-ethenyl, ω-(2,2-Dichlor-1-methylcyclopropyl)-$C_{1-4}$-alkoxy und (2,2-Dichlor-1-methyl-cyclopropyl)-carbonyl.

**11.** Verfahren nach Anspruch 1, in welchem die folgenden Verbindungen hergestellt werden: 2-tert.-Butyl-4-chlor-5-[4-(1,1,2-trifluor-2-trifluormethoxyethoxy)-α-methylbenzyloxy]-3(2H)-pyridazinon, 2-tert.-Butyl-4-chlor-5-[4-(1,1,2-trifluor-2-trifluormethoxyethoxy)-α-methylbenzylthio]-3(2H)-pyridazinon, 2-tert.-Butyl-4-chlor-5-[4-(1,1,2-trifluor-2-trifluormethoxyethoxy)-benzyloxy]-3(2H)-pyridazinon, 2-tert.-Butyl-4-chlor-5-[4-(1,1,2-trifluor-2-trifluormethoxyethoxy)-benzylthio]-3(2H)-pyridazinon,2-tert.-Butyl-4-chlor-5-[4-(2-pentafluorethoxy-1,1,2-trifluorethoxy)-benzylthio]-3(2H)-pyridazinon, 2-tert.-Butyl-4-chlor-5[4-(2-pentafluor-ethoxy-1,1,2-trifluorethoxy)-α-methylbenzylthio]-3(2H)-pyridazinon, 2-tert.-Butyl-4-chlor-5-[4-(2-chlor-3,3,3-trifluorprope nyl)-benzylthio]-3(2H)-pyridazinon, 2-tert.-Butyl-4-chlor-5-[4-(2,2-dichlorethenyl)-benzylthio]-3(2H)-pyridazinon, 2-tert.-Butyl-4-chlor-5-[4-(1,1,2-trifluor-2-trifluormethoxyethoxyethyl)-benzylthio]-3(2H)-pyridazinon,2-tert.-Butyl-4-chlor-5-[4-(1,2-difluor-2-trifluormethoxyethenyl)-benzylthio]-3(2H)-pyridazinon, 2-tert.-Butyl-4-chlor-5-[4-(2-chlor-3,3,3-trifluorpropenyl)-α-methylbenzylthio]-3(2H)-pyridazinon, 2-tert.-Butyl-4-chlor-5-[4-(2,2-dichlorethenyl)-α-methyl-benzylthio]-3(2H)-pyridazinon, 2-tert.-Butyl-4-chlor-5-[4-(1,2-difluor-2-trifluormethoxyethenyl)-α-methylbenzylthio]-3(2H)-pyridazinon, 2-tert.-Butyl-4-chlor-5-[4-(2-chlor-2-phenylethenyl)-benzylthio]-3(2H)-pyridazinon, 2-tert.-Butyl-4-chlor-5-[4-(2-chlor(4-chlorphenylethenyl)-benzylthio]-3(2H)-pyridazinon, 2-tert.-Butyl-4-chlor-5-[4-(2,2-dichlor-1-methyl-cyclopropylmethoxy)-benzylthio]-3(2H)-pyridazinon, 2-tert.-Butyl-4-chlor-5-[4-(1,2-dichlor1-methylcyclopropylcarbonyl)-benzylthio]-3(2H)-pyridazinon,2-(3,4-Dichlorphenyl)-4-chlor-5-[4-(1,1,2-trifluor2-triflu or-methoxyethoxy)-benzyloxy]-3(2H)-pyridazinon und 2-(3,4-Dichlorphenyl)-4-chlor-5-[4-(1,1,2-trifluor2-trifluormethoxyethoxy)-benzylthio]-3(2H)-pyridazinon.

**12.** Verfahren zur Kontrolle des Befalls durch schädliche Milben und/oder Insekten im landwirtschaftlichen Anbau, in Gartenbau-Kulturen und/oder anderen Bereichen, umfassend die Anwendung mindestens eines Pyridazinons nach irgendeinem der Ansprüche 1 bis 11 auf den Anbau und/oder den Bereich.

**13.** Zusammensetzungen zur Kontrolle des Befalls durch schädliche Milben und/oder Insekten, umfassend mindestens ein Pyridazinon gemäß irgendeinem der Ansprüche 1 bis 11, einen oder mehrere feste oder flüssige Trägerstoffe und gegebenenfalls weitere übliche Additive und/oder aktive Bestandteile und/oder Düngemittel.

**Revendications**

**Revendications pour les Etats contractants suivants : AT, BE, CH, DE, FR, GB, LI, NL**

**1.** Pyridazinones de formule générale (I) :

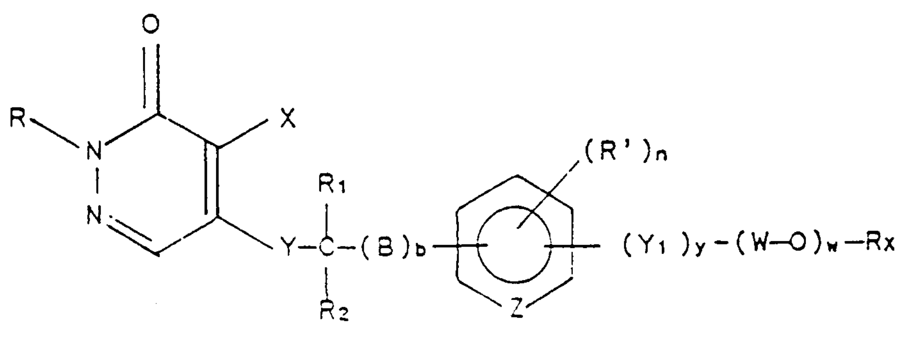

(I)

dans laquelle :

| | |
|---|---|
| R | représente un groupe alkyle en $C_{2-6}$ à chaîne droite ou ramifiée; ou un groupe phényle, éventuellement substitué avec des atomes d'halogène et/ou des groupes alkyles en $C_{1-6}$, haloalkyles en $C_{1-6}$, alcoxy en $C_{1-6}$ et/ou haloalcoxy en $C_{1-6}$; |
| X | représente un atome d'halogène; |
| Y | représente un atome d'oxygène ou de soufre ou un radical NH; |
| B | représente un groupe |

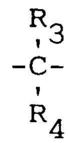

b est 0 ou 1;

$R_1$, $R_2$, $R_3$ et $R_4$ représentent indépendamment un atome d'hydrogène ou un groupe alkyle en $C_{1-4}$ ou haloalkyle en $C_{1-6}$ à chaîne droite ou ramifiée;

Z représente un radical CH ou un atome d'azote;

$Y_1$ représente un atome d'oxygène ou de soufre ou un radical CO, ou

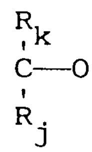

dans lequel :

$R_k$ et $R_j$ représentent indépendamment un atome d'hydrogène ou un groupe alkyle en $C_{1-4}$ ou haloalkyle en $C_{1-6}$ à chaîne droite ou ramifiée;

W est un groupe haloalkylène en $C_{2-6}$ à chaîne droite ou ramifiée;

y et w sont indépendamment 0 ou 1;

$R_x$ lorsque w = 0, représente un groupe haloalcényle en $C_{2-6}$ à chaîne droite ou ramifiée, ayant éventuellement un groupe haloalcoxy en $C_{1-3}$ ou phényle comme substituant, ce groupe phényle étant à son tour éventuellement substitué avec un ou plusieurs atomes d'halogène et/ou groupes alkyle en $C_{1-6}$, haloalkyle en $C_{1-6}$, alcoxy en $C_{1-6}$ et/ou haloalcoxy en $C_{1-6}$; ou représente un groupe halocycloalkyle en $C_{3-6}$ ou halocycloalkylalkyle en $C_{4-7}$, lesquels groupes peuvent avoir un ou plusieurs substituants choisis parmi les groupes alkyle en $C_{1-4}$ ou haloalkyle en $C_{1-6}$; et
lorsque w = 1, $R_x$ représente un groupe haloalkyle en $C_{1-6}$ à chaîne droite ou ramifiée;

R' représente un atome d'halogène; et

n est 0, 1 ou 2.

**2.** Composés selon la revendication 1, ayant la formule générale (Ia) :

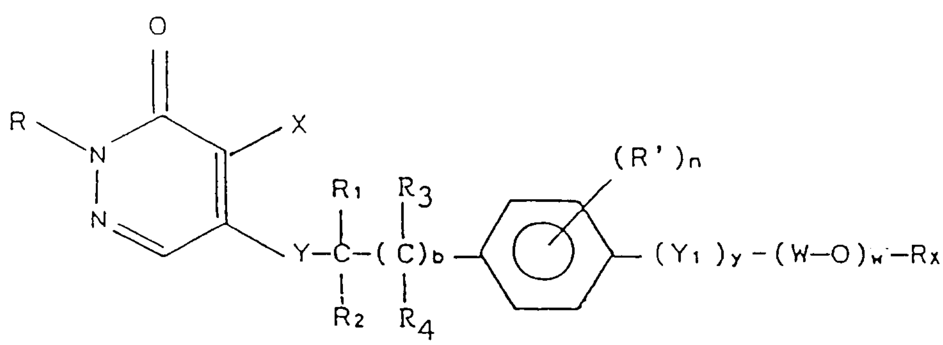

(Ia)

dans laquelle :

R représente un groupe alkyle en $C_{2-6}$;

Y représente un atome d'oxygène ou de soufre;

et les autres groupes sont tels que définis dans la revendication 1.

3. Composés selon l'une quelconque des revendications 1 et 2, dans lesquels R est un groupe butyle ou un groupe phényle éventuellement substitué.

4. Composés selon la revendication 3, dans lesquels R est un groupe t-butyle ou un groupe phényle substitué par un atome de chlore et/ou de fluor.

5. Composés selon l'une quelconque des revendications 1 à 4, dans lesquels X est un atome de F, Cl ou Br.

6. Composés selon la revendication 5, dans lesquels X est un atome de Cl.

7. Composés selon l'une quelconque des revendications 1 à 6, dans lesquels $R_1$ est un atome d'hydrogène ou un groupe alkyle en $C_{1-4}$, $R_2$ est un atome d'hydrogène et b est 0.

8. Composés selon la revendication 7, dans lesquels $R_1$ est un groupe méthyle.

9. Composés selon l'une quelconque des revendications 1 à 8, dans lesquels la partie $-(Y_1)_y-(W-O)_w-R_x$ contient au moins un atome de F et/ou de Cl.

10. Composés selon la revendication 9, dans lesquels la partie $-(Y_1)_y-(W-O)_w-R_x$ est choisie parmi des groupes 1,1,2-trifluoro-2-(alcoxy en $C_{1-4}$ partiellement ou complètement fluoré)-éthoxy, 2-chloro-2-(alkyle en $C_{1-4}$ partiellement ou complètement fluoré)-éthényle, 2,2-dichloro-éthényle, 1,2-difluoro-2-(alcoxy en $C_{1-4}$ partiellement ou complètement fluoré)-éthényle, 2-chloro-2-(phényle éventuellement substitué)-éthényle, w-(2,2-dichloro-1-méthyl-cyclopropyl)-(alcoxy en $C_{1-4}$) et (2,2-dichloro-1-méthyl-cyclopropyl)-carbonyle.

11. Composés selon la revendication 1, c'est-à-dire:
   . 2-t-butyl-4-chloro-5-[4-(1,1,2-trifluoro-2-trifluorométhoxy-éthoxy)-α-méthyl-benzyloxy]-3(2H)-pyridazinone,
   . 2-t-butyl-4-chloro-5-[4-(1,1,2-trifluoro-2-trifluorométhoxy-éthoxy)-α-méthyl-benzylthio]-3(2H)-pyridazinone,
   . 2-t-butyl-4-chloro-5-[4-(1,1,2-trifluoro-2-trifluorométhoxy-éthoxy)-benzyloxy]-3(2H)-pyridazinone,
   . 2-t-butyl-4-chloro-5-[4-(1,1,2-trifluoro-2-trifluorométhoxy-éthoxy)-benzylthio]-3(2H)-pyridazinone,
   . 2-t-butyl-4-chloro-5-[4-(2-pentafluoroéthoxy-1,1,2-trifluoroéthoxy)-benzylthio]-3(2H)-pyridazinone,
   . 2-t-butyl-4-chloro-5-[4-(2-pentafluoroéthoxy-1,1,2-trifluoroéthoxy)-α-méthyl-benzylthio]-3(2H)-pyridazinone,
   . 2-t-butyl-4-chloro-5-[4-(2-chloro-3,3,3-trifluoropropényl)-benzylthio]-3(2H)-pyridazinone,
   . 2-t-butyl-4-chloro-5-[4-(2,2-dichloro-éthényl)-benzylthio]-3(2H)-pyridazinone,
   . 2-t-butyl-4-chloro-5-[4-(1,1,2-trifluoro-2-trifluorométhoxy-éthoxy-éthyl)-benzylthio]-3(2H)-pyridazinone,
   . 2-t-butyl-4-chloro-5-[4-(1,2-difluoro-2-trifluorométhoxyéthényl)-benzylthio]-3(2H)-pyridazinone,
   . 2-t-butyl-4-chloro-5-[4-(2-chloro-3,3,3-trifluoropropényl)-α-méthyl-benzylthio]-3(2H)-pyridazinone,
   . 2-t-butyl-4-chloro-5-[4-(2,2-dichloro-éthényl)-α-méthylbenzylthio]-3(2H)-pyridazinone,
   . 2-t-butyl-4-chloro-5-[4-(1,2-difluoro-2-trifluorométhoxy-éthényl)-α-méthyl-benzylthio]-3(2H)-pyridazinone,
   . 2-t-butyl-4-chloro-5-[4-(2-chloro-2-phényl-éthényl)-benzylthio]-3(2H)-pyridazinone,
   . 2-t-butyl-4-chloro-5-[4-(2-chloro-(4-chlorophényl-éthényl) -benzylthio]-3(2H)-pyridazinone,
   . 2-t-butyl-4-chloro-5-[4-(2,2-dichloro-1-méthyl-cyclopropyl -méthoxy)-benzylthio]-3(2H)-pyridazinone,
   . 2-t-butyl-4-chloro-5-[4-(2,2-dichloro-1-méthyl-cyclopropyl -carbonyl)-benzylthio]-3(2H)-pyridazinone,
   . 2-(3,4-dichlorophényl)-4-chloro-5-[4-(1,1,2-trifluoro-2-trifluorométhoxy-éthoxy)-benzyloxy]-3(2H)-pyridazinone, et
   . 2-(3,4-dichlorophényl)-4-chloro-5-[4-(1,1,2-trifluoro-2-trifluorométhoxy-éthoxy)-benzylthio]-3(2H)-pyridazinone.

12. Procédé de préparation des composés selon la revendication 1, caractérisé en ce qu'on fait réagir une pyridazinone de formule (II):

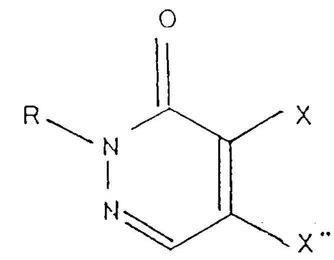

(II)

avec un composé de formule (III):

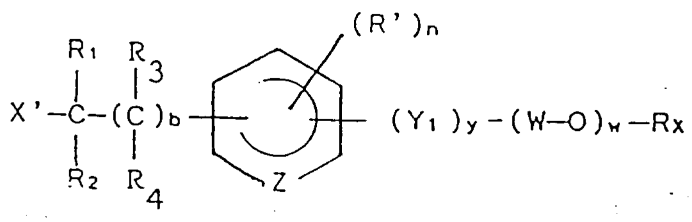

(III)

dans lesquelles les symboles R, X, $R_1$ $R_2$, $R_3$, $R_4$, Z, $Y_1$, W, $R_x$, R', b, y, w et n sont tels que définis dans la revendication 1 et les symboles X' et X" représentent des atomes de Cl, Br ou I ou un radical YH, Y étant tel que défini dans la revendication 1, à condition que l'un des X' et X" soit un radical YH alors que l'autre est un atome de Cl, Br ou I;
dans un solvant organique, en présence d'une base inorganique ou organique et à une température comprise entre la température ambiante et la température de reflux du mélange réactionnel.

**13.** Méthode pour lutter contre des infestations par des acariens et/ou des insectes nuisibles des cultures agricoles, des cultures horticoles et/ou d'autres lieux, comprenant l'application d'au moins une pyridazinone selon l'une quelconque des revendications 1 à 11 sur cette culture et/ou ce lieu.

**14.** Compositions pour lutter contre des infestations par des acariens et/ou des insectes nuisibles, comprenant au moins une pyridazinone selon l'une quelconque des revendications 1 à 11, un ou plusieurs supports solides ou liquides et éventuellement, d'autres additifs habituels et/ou composants actifs et/ou engrais.

**Revendications pour l'Etat contractant suivant : ES**

**1.** Procédé pour la préparation des pyridazinones de formule générale (I) :

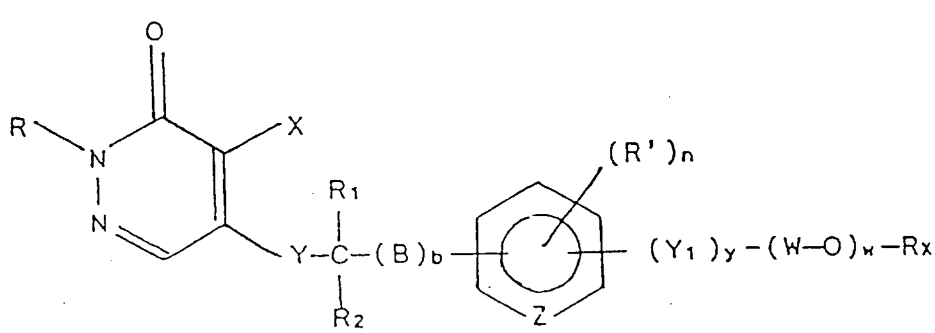

(I)

dans laquelle :

| | |
|---|---|
| R | représente un groupe alkyle en $C_{2-6}$ à chaîne droite ou ramifiée; ou un groupe phényle, éventuellement substitué avec des atomes d'halogène et/ou des groupes alkyles en $C_{1-6}$, haloalkyles en $C_{1-6}$, alcoxy en $C_{1-6}$ et/ou haloalcoxy en $C_{1-6}$; |

X représente un atome d'halogène;

Y représente un atome d'oxygène ou de soufre ou un radical NH;

B représente un groupe

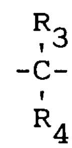

b est 0 ou 1;

$R_1$, $R_2$, $R_3$ et $R_4$ représentent indépendamment un atome d'hydrogène ou un groupe alkyle en $C_{1-4}$ ou haloalkyle en $C_{1-6}$ à chaîne droite ou ramifiée;

Z représente un radical CH ou un atome d'azote;

$Y_1$ représente un atome d'oxygène ou de soufre ou un radical CO, ou

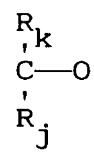

dans lequel :

$R_k$ et $R_j$ représentent indépendamment un atome d'hydrogène ou un groupe alkyle en $C_{1-4}$ ou haloalkyle en $C_{1-6}$ à chaîne droite ou ramifiée;

W est un groupe haloalkylène en $C_{2-6}$ à chaîne droite ou ramifiée;

y et w sont indépendamment 0 ou 1;

$R_x$ lorsque w = 0, représente un groupe haloalcényle en $C_{2-6}$ à chaîne droite ou ramifiée, ayant éventuellement un groupe haloalcoxy en $C_{1-3}$ ou phényle comme substituant, ce groupe phényle étant à son tour éventuellement substitué avec un ou plusieurs atomes d'halogène et/ou groupes alkyles en $C_{1-6}$, haloalkyles en $C_{1-6}$, alcoxy en $C_{1-6}$ et/ou haloalcoxy en $C_{1-6}$; ou représente un groupe halocycloalkyle en $C_{3-6}$ ou halocycloalkylalkyle en $C_{4-7}$, lesquels groupes peuvent avoir un ou plusieurs substituants choisis parmi des groupes alkyles en $C_{1-4}$ ou haloalkyles en $C_{1-6}$; et

lorsque w = 1, $R_x$ représente un groupe haloalkyle en $C_{1-6}$ à chaîne droite ou ramifiée;

R' représente un atome d'halogène; et

n est 0, 1 ou 2,

caractérisé que ce qu'on fait réagir une pyridazinone de formule (II):

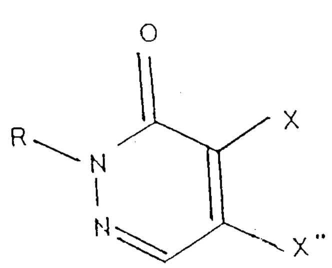

(II)

avec un composé de formule (III) :

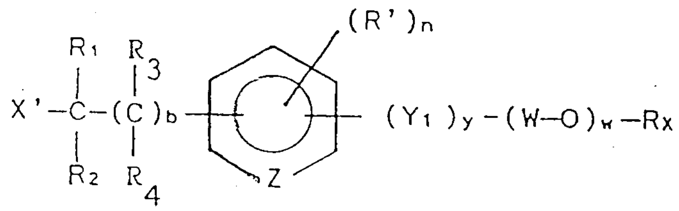

(III)

dans lesquelles les symboles R, X, $R_1$, $R_2$, $R_3$, $R_4$, Z, $Y_1$, W, $R_x$, R', b, y, w et n sont tels que définis plus haut et les symboles X' et X'' représentent des atomes de Cl, Br ou I ou un radical YH, Y étant tel que défini plus haut, à condition que l'un des X' et X'' soit un radical YH alors que l'autre est un atome de Cl, Br ou I;
dans un solvant organique, en présence d'une base inorganique ou organique et à une température comprise entre la température ambiante et la température de reflux du mélange réactionnel.

**2.** Procédé selon la revendication 1, caractérisé en ce que les composés ayant la formule générale (Ia):

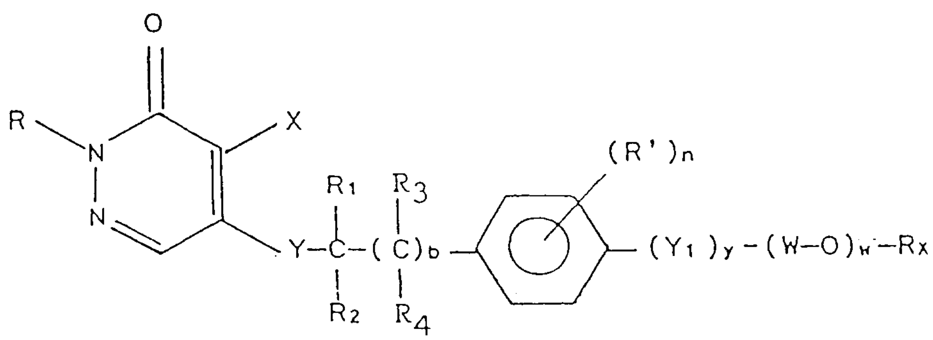

(Ia)

dans laquelle :
R représente un groupe alkyle en $C_{2-6}$;
Y représente un atome d'oxygène ou de soufre;
et les autres groupes sont tels que définis dans la revendication 1, sont préparés.

**3.** Procédé selon l'une quelconque des revendications 1, et 2, dans lequel R est un groupe butyle ou un groupe phényle éventuellement substitué.

**4.** Procédé selon la revendication 3, dans lequel R est un groupe t-butyle ou un groupe phényle substitué par un atome de chlore et/ou de fluor.

**5.** Procédé selon l'une quelconque des revendications 1 à 4, dans lequel X est un atome de F, Cl ou Br.

**6.** Procédé selon la revendication 5, dans lequel X est un atome de Cl.

**7.** Procédé selon l'une quelconque des revendications 1 à 6, dans lequel $R_1$ est un atome d'hydrogène ou un groupe alkyle en $C_{1-4}$, $R_2$ est un atome d'hydrogène et b est 0.

**8.** Procédé selon la revendication 7, dans lequel $R_1$ est un groupe méthyle.

**9.** Procédé selon l'une quelconque des revendications 1 à 8, dans lequel la partie $-(Y_1)_y-(W-O)_w-R_x$ contient au moins un atome de F et/ou de Cl.

**10.** Procédé selon la revendication 9, dans lequel la partie $-(Y_1)_y-(W-O)_w-R_x$ est choisie parmi des groupes 1,1,2-trifluoro-2-(alcoxy en $C_{1-4}$ partiellement ou complètement fluoré)-éthoxy, 2-chloro-2-(alkyle en $C_{1-4}$ partiellement ou complètement fluoré)-éthényle, 2,2-dichloro-éthényle, 1,2-difluoro-2-(alcoxy en $C_{1-4}$ partiellement ou complètement fluoré)-éthényle, 2-chloro-2-(phényle éventuellement substitué)-éthényle, w-(2,2-dichloro-1-méthyl-cyclopropyl)-(alcoxy en $C_{1-4}$) et (2,2-dichloro-1-méthyl-cyclopropyl)-carbonyle.

**11.** Procédé selon la revendication 1, pour la préparation des composés suivants:
. 2-t-butyl-4-chloro-5-[4-(1,1,2-trifluoro-2-trifluorométhoxy-éthoxy)-α-méthyl-benzyloxy]-3(2H)-pyridazinone,
. 2-t-butyl-4-chloro-5-[4-(1,1,2-trifluoro-2-trifluorométhoxy-éthoxy)-α-méthyl-benzylthio]-3(2H)-pyridazinone,
. 2-t-butyl-4-chloro-5-[4-(1,1,2-trifluoro-2-trifluorométhoxy-éthoxy)-benzyloxy]-3(2H)-pyridazinone,
. 2-t-butyl-4-chloro-5-[4-(1,1,2-trifluoro-2-trifluorométhoxy-éthoxy)-benzylthio]-3(2H)-pyridazinone,
. 2-t-butyl-4-chloro-5-[4-(2-pentafluoroéthoxy-1,1,2-trifluoroéthoxy)-benzylthio]-3(2H)-pyridazinone,
. 2-t-butyl-4-chloro-5-[4-(2-pentafluoroéthoxy-1,1,2-trifluoroéthoxy)-α-méthyl-benzylthio]-3(2H)-pyridazinone,
. 2-t-butyl-4-chloro-5-[4-(2-chloro-3,3,3-trifluoropropényl)-benzylthio]-3(2H)-pyridazinone,
. 2-t-butyl-4-chloro-5-[4-(2,2-dichloro-éthényl)-benzylthio]-3(2H)-pyridazinone,
. 2-t-butyl-4-chloro-5-[4-(1,1,2-trifluoro-2-trifluorométhoxyéthoxy-éthyl)-benzylthio]-3(2H)-pyridazinone,
. 2-t-butyl-4-chloro-5-[4-(1,2-difluoro-2-trifluorométhoxyéthényl)-benzylthio]-3(2H)-pyridazinone,
. 2-t-butyl-4-chloro-5-[4-(2-chloro-3,3,3-trifluoropropényl)-α-méthyl-benzylthio]-3(2H)-pyridazinone,
. 2-t-butyl-4-chloro-5-[4-(2,2-dichloro-éthényl)-α-méthylbenzylthio]-3(2H)-pyridazinone,
. 2-t-butyl-4-chloro-5-[4-(1,2-difluoro-2-trifluorométhoxyéthényl)-α-méthyl-benzylthio]-3(2H)-pyridazinone,
. 2-t-butyl-4-chloro-5-[4-(2-chloro-2-phényl-éthényl)-benzylthio]-3(2H)-pyridazinone,
. 2-t-butyl-4-chloro-5-[4-(2-chloro-(4-chlorophényl-éthényl) -benzylthio]-3(2H)-pyridazinone,
. 2-t-butyl-4-chloro-5-[4-(2,2-dichloro-1-méthyl-cyclopropyl -méthoxy)-benzylthio]-3(2H)-pyridazinone,
. 2-t-butyl-4-chloro-5-[4-(2,2-dichloro-1-méthyl-cyclopropyl -carbonyl)-benzylthio]-3(2H)-pyridazinone,
. 2-(3,4-dichlorophényl)-4-chloro-5-[4-(1,1,2-trifluoro-2-trifluorométhoxy-éthoxy)-benzyloxy]-3(2H)-pyridazinone, et
. 2-(3,4-dichlorophényl)-4-chloro-5-[4-(1,1,2-trifluoro-2-trifluorométhoxy-éthoxy)-benzylthio]-3(2H)-pyridazinone.

**12.** Méthode pour lutter contre des infestations par des acariens et/ou des insectes nuisibles des cultures agricoles, des cultures horticoles et/ou d'autres lieux, comprenant l'application d'au moins une pyridazinone suivant l'une quelconque des revendications 1 à 11 sur cette culture et/ou ce lieu.

**13.** Compositions pour lutter contre des infestations par des acariens et/ou des insectes nuisibles, comprenant au moins une pyridazinone suivant l'une quelconque des revendications 1 à 11, un ou plusieurs supports solides ou liquides et éventuellement, d'autres additifs habituels et/ou composants actifs et/ou engrais.